# EUROPEAN PATENT SPECIFICATION

(11) **EP 4 023 271 B1**
(45) Date of publication and mention of the grant of the patent: **11.09.2024**
(21) Application number: 20306706.1
(22) Date of filing: 29.12.2020
(51) Int. Cl.: A61M 39/10, A61M 1/36

(54) **DISPOSABLE CIRCUIT FOR EXTRACORPOREAL TREATMENT OF BLOOD, APPARATUS FOR EXTRACORPOREAL TREATMENT OF BLOOD AND ASSOCIATED METHOD**
EINWEGSCHALTUNG ZUR EXTRAKORPORALEN BEHANDLUNG VON BLUT, GERÄT ZUR EXTRAKORPORALEN BEHANDLUNG VON BLUT UND ZUGEHÖRIGES VERFAHREN
CIRCUIT JETABLE POUR LE TRAITEMENT EXTRACORPOREL DU SANG, APPAREIL POUR LE TRAITEMENT EXTRACORPOREL DU SANG ET PROCÉDÉ ASSOCIÉ

(43) Date of publication of application: 06.07.2022
(73) Proprietor: Gambro Lundia AB, 226 43 Lund (SE)
(72) Inventor: POUCHOULIN, Dominique, 01390 Tramoyes (FR)
(74) Representative: Ponzellini, Gian-Marco

(56) References cited:
- US-A- 5 630 946
- US-A- 5 895 368
- US-A1- 2009 221 949
- US-B2- 6 913 588

## Description

### FIELD OF THE INVENTION

The present disclosure relates to a disposable circuit for extracorporeal treatment of blood configurable for fluid recirculation, to an apparatus for extracorporeal treatment of blood, a method of blood flow recirculation in the disposable circuit and an associated computer program product.

The disposable circuit is particularly suitable for temporary disconnection of a patient from the apparatus for extracorporeal treatment of blood and subsequent reconnection to the same disposable circuit for treatment prosecution.

### BACKGROUND ART

Extracorporeal blood treatment involves removing blood from a patient, treating the blood externally to the patient, and returning the treated blood to the patient. Extracorporeal blood treatment is typically used to extract undesirable matter or molecules from the patient's blood and/or add desirable matter or molecules to the blood. Extracorporeal blood treatment is used with patients unable to effectively remove matter from their blood, such as when a patient has suffered temporary or permanent kidney failure. These patients and other patients may undergo extracorporeal blood treatment to add or remove matter to their blood, to maintain an acid/base balance or to remove excess body fluids, for example.

Extracorporeal blood treatment is typically accomplished by removing the blood from the patient in e.g. a continuous flow, introducing the blood into a primary chamber, also referred to as blood chamber, of a filtration unit (such as a dialyzer or a hemofilter) where the blood is allowed to flow past a semipermeable membrane. The semipermeable membrane selectively allows matter in the blood to cross the membrane from the primary chamber into a secondary chamber and also selectively allows matter in the secondary chamber to cross the membrane into the blood in the primary chamber, depending on the type of treatment.

A number of different types of extracorporeal blood treatments may be performed. In an ultrafiltration (UF) treatment, undesirable matter is removed from the blood by convection across the membrane into the secondary chamber. In a hemofiltration (HF) treatment, the blood flows past the semipermeable membrane as in UF and desirable matter is added to the blood, typically by dispensing a fluid into the blood either before and/or after it passes through the filtration unit and before it is returned to the patient. In a hemodialysis (HD) treatment, a secondary fluid containing desirable matter is introduced into the secondary chamber of the filtration unit. Undesirable matter from the blood crosses the semipermeable membrane into the secondary fluid and desirable matter from the secondary fluid may cross the membrane into the blood. In a hemodiafiltration (HDF) treatment, blood and secondary fluid exchange matter as in HD, and, in addition, matter is added to the blood, typically by dispensing a fluid into the treated blood before its return to the patient as in HF.

Specific blood treatment apparatuses have been developed for the treatment of acute patients mainly because:
- in acute patients, the total treatment time is a priori unknown and, as such, it may not be used as setup parameter as it is not known how long kidney insufficiency will be present; indeed, acute patients often need relatively long treatment sessions, typically lasting several days, before recovering from kidney failure;
- intensive care apparatus may rather be designed to request a plurality of flow rate information as setup parameters;
- acute patients need a very precisely controlled fluid removal; moreover, infusion of drugs or of fluids in general shall also be very accurately controlled;
- acute patients are also very weak; thus, in the course of the treatment of acute patients it has been regarded as relevant to make sure that a continuous and gentle therapy is delivered to the patient during treatment with a very accurate control on the flow rate in each of the lines connected with the bloodlines;
- furthermore, apparatus often need to be operated in emergency situations, thus apparatus for acute treatment shall be characterized by very easy to set up procedures.

Some treatments for patients may involve very long procedures wherein blood is treated for several days or even weeks; this is the case of continuous renal replacement therapies CRRT wherein the blood treatment apparatuses may be operated continuously for some days. Irrespective of whether the treatment is for continuous renal replacement, or for acute or chronic patients, there may be cases wherein the patient may need to be disconnected from the apparatus when the treatment is still not yet completed. This is the case for instance when the patient shall be subjected to medical exams to be performed outside the renal unit room or when an intervention on the vascular access shall be suddenly performed. Disconnecting and reconnecting the blood treatment apparatus to the blood circuit lines requires disconnecting these lines from the patient, in particular from a venous connector and an arterial connector arranged on the ends of the blood circuit apparatus (for example the patient is disconnected from respective venous and arterial needles inserted into the fistula - chronic treatments - or from a catheter fixed to the patient - acute treatments).

Such temporary disconnection is critical at least for the following reasons. Disconnection and reconnection of blood circuit of the blood treatment apparatuses shall be performed in sterile conditions, and this requires a qualified operator who may not interact with the control unit of the apparatus, which is typically not sterile. Stagnant blood in the blood circuit of the apparatus may coagulate; therefore, the blood treatment apparatus shall be left non-operative for very short times to avoid harming the patient safety. On the other hand, complete substitution of disposable circuits during temporary interruption of the therapy should be avoided at least for an economic reason; in this latter case, substitution of disposable circuits would imply significantly higher costs for each single treatment.

Document US9713670 discloses a method for temporarily interrupting an extracorporeal treatment of blood of a patient by use of a blood treatment apparatus. The method comprises activating a control device configured to bring the blood treatment apparatus into a state in which the blood treatment session of the patient can be interrupted with the intention to continue the blood treatment session. The ends of the blood lines are disconnected from the patient and connected through an 'Y' connector to the same saline solution bag. The fluid is then recirculated inside the blood circuit.

Document US2009/221949 describes a device for circulatory isolation and treatment of a part of a patient body. A pump is aspirating liquid from a venous reservoir for sending it to a heater and oxygenator unit. An outlet of the oxygenator unit is connected to a first arterial Y-connector. The mainstream outlet of the first arterial Y-connector is connected via an arterial trunk conduit to the patient's arterial catheter. On the venous side, blood or liquid coming from the patient via a venous catheter passes a venous front conduit and a venous Y-connector. A shunt inlet of the venous Y-connector is connected to a shunting outlet of the first arterial Y-connector by means of a shunt conduit. The mainstream outlet is connected to the venous reservoir with an integrated filter. A third port of connector may send liquid to a collecting bag.

Document US6913588 shows an extracorporeal circuit connected in line with an existing hemofiltration system. The hemofiltration system emerges from a patient venous connection in a conduit provided with a blood pump. An anticoagulant and replacement fluids are infused into the conduit with a pump to prevent blood coagulation and replace blood volume lost as a waste product. Blood enters a hemofilter where ultrafiltrate is separated from the blood. After filtration, blood leaves the hemofilter along a bloodline, through a shunt and a conduit, returning to a patient arterial system. Ultrafiltrate leaves the hemofilter at an ultrafiltrate port through the ultrafiltrate conduit.

Document US5630946 discloses a method for removing leukocytes from the blood in an extracorporeal circuit by passing a leukocyte containing blood from a patient into a container, passing a portion of biological fluid from the container through a recirculation loop including a filter assembly for removing leukocytes and moving the portion of leukocyte-depleted blood into the container; additional blood with leukocyte is transferred from a patient into the container so that the additional blood is mixed with leukocyte depleted blood in the container to provide a mixture and then a portion of the mixture is sent to the patient while passing another portion of the mixture through the recirculation loop.

Document US5895368 discloses an extracorporeal blood set for use in hemodialysis. The set comprises an arterial line connected to a dialyzer and from there to a venous set. Arterial line has a patient connector and a tubing with a roller pump tubing and distal end connector for connection with dialyzer. T-connector carries a branching tube which comprises an integrally and permanently attached set for connecting at its remote end to a source of saline. Branching solution set carries a Y member, to which is connected further branch tubing, which is terminated with a female luer lock connector.

It is an object of embodiments of the present disclosure to provide a disposable circuit for extracorporeal treatment of blood particularly suitable for intensive care, but also for chronic care applications which helps a rapid and safe temporary disconnection of the patient from the apparatus during the execution of a blood treatment. Furthermore, it is an object of embodiments of the disclosure a disposable circuit for extracorporeal treatment of blood, which is economically convenient to use. It is a further object of embodiments of the disclosure a disposable circuit for extracorporeal treatment of blood, which prevents errors of fluid management for the fluids still resting in the apparatus while the patient is temporarily disconnected therefrom. It is a further object of embodiments of the disclosure a disposable circuit for extracorporeal treatment of blood which allows a safe reconnection of the patient to the apparatus, and a safe reintroduction of the fluid and/or blood still present in the apparatus into the circulatory apparatus of the patient when this latter is reconnected to the treatment machine. It is a further object of embodiments of the disclosure to provide a disposable circuit that, when the patient is disconnected from the apparatus, is configurable in a configuration which prevents stagnation and/or reduces the risk of coagulation of the blood still present therein.

The invention is defined by the technical features of independent claims 1, 2 and 15.

### DESCRIPTION OF THE DRAWINGS

Aspects of the invention are shown in the attached drawings, which are provided by way of non-limiting example, wherein:
- Figure 1 shows a general schematic diagram of a disposable circuit according to an embodiment of the invention,
- Figure 2 and Figure 3 show exemplary type of connection of auxiliary connectors of the disposable circuit according to an embodiment of the invention, when such auxiliary connectors are arranged on a pre-blood pump pre-dilution infusion line;
- Figure 4 and Figure 5 show exemplary type of connection of auxiliary connectors of the disposable circuit according to an embodiment of the invention, when such auxiliary connectors are arranged on a post-blood pump pre-dilution infusion line;
- Figure 6 shows a specific and partial schematic diagram of a configuration of the disposable circuit;
- Figure 7 and Figure 8 show exemplary type of connection of auxiliary connectors of the disposable circuit according to an embodiment of the invention, when such auxiliary connectors are arranged on a post-dilution fluid line;
- Figure 9 shows a specific and partial schematic diagram of a configuration of the disposable circuit;
- Figure 10 and Figure 11 show alternative types of connection of auxiliary connectors of the disposable circuit according to an embodiment of the invention, when such auxiliary connectors are arranged on a blood circuit;
- Figure 12, 13, and 14 show exemplary type of connection of auxiliary connectors of the disposable circuit according to an embodiment of the invention, when such auxiliary connectors are arranged on one, or two, auxiliary lines;
- figures 15-19 show alternative configurations for auxiliary connectors of the disposable circuit;

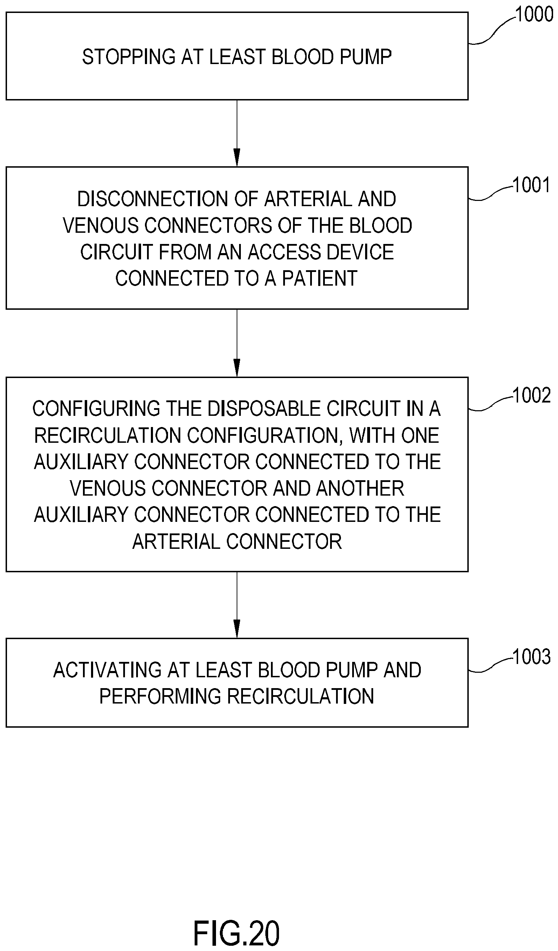

- figure 20 shows a flow chart of some operative steps required for switching from a treatment configuration to a recirculation configuration for the apparatus and disposable circuit object of the present disclosure.

### DETAILED DESCRIPTION

Non-limiting embodiments of an apparatus 1 for extracorporeal treatment of blood - which may implement innovative aspects of the invention - are shown in figures. In below description and in figures, same components are identified by same reference numerals.

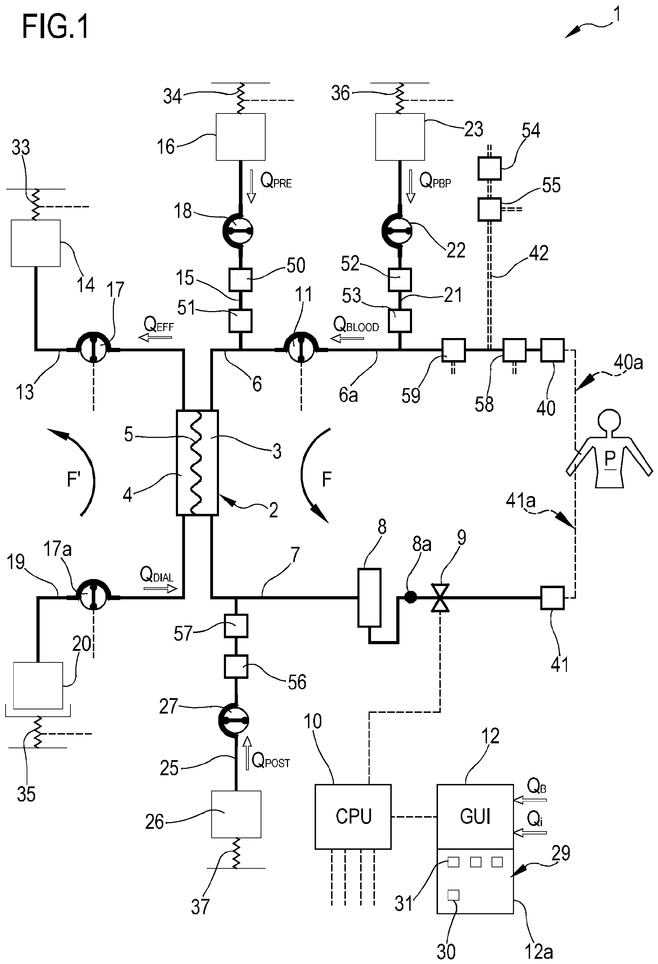

Figure 1 shows an apparatus 1 designed for delivering extracorporeal blood treatments to patients, such as treatment for removing unwanted matter from the blood thereof.

According to further embodiments, an apparatus for extracorporeal blood treatment by hemoperfusion and/or or ExtraCorporeal CO2 Removal (ECCO2R) are also included in the scope of the invention.

In a non-limiting embodiment, the apparatus 1 is conceived for running of at least one of the following treatments: hemodialysis (HD), hemofiltration with pre-dilution (HFₚᵣₑ), hemofiltration with post-dilution (HFₚₒₛₜ), hemofiltration with both pre-dilution and post-dilution (HFₚᵣₑ₋ₚₒₛₜ), hemodiafiltration with pre-dilution (HDFₚᵣₑ), hemodiafiltration with post-dilution (HDFₚₒₛₜ), hemodiafiltration with both pre-dilution and post-dilution (HDFₚᵣₑ₋ₚₒₛₜ), ultrafiltration (UF). The apparatus may alternatively provide plasmafiltration.

The apparatus 1 comprises a disposable circuit for blood treatment, which comprises several elements hereinafter identified. For the purposes of the present invention, as per "disposable" shall be meant an element intended, specifically conceived, for being thrown away or anyway disposed after use, in particular after a single use. In particular it shall be noted that for "single use" shall be intended a single patient treatment. It is understood that the temporary disconnection of the patient from the apparatus 1, in the course of a non-completed treatment, does not interrupt the single treatment; reconnection of the patient to the disposable circuit after the temporary disconnection is yet still within the single use as herewith defined. The elements which according to any of the non-limiting embodiments hereinafter described are part of the disposable circuit, are marked in thick lines in the annexed figures; those being part of the apparatus 1 are marked in thin lines; this helps the reader to clearly identify which elements belong to the disposable circuit and which are in contrast part of the apparatus. The disposable circuit hereinafter described may be detachably connected to the apparatus and may be sold or anyway provided separately from the apparatus 1.

The disposable circuit comprises a filtration unit 2, such as an hemofilter, an ultrafilter, an hemodiafilter, a dialyzer, a plasmafilter, a gas exchanger, an adsorbent cartridge and the like; specifically, in a first example according to figure 1 particularly dealing with a dialysis apparatus, the filtration unit may have a primary chamber 3 and a secondary chamber 4 separated by a semi-permeable membrane 5; depending upon the treatment the membrane of the filtration unit may be selected to have different properties and performances.

The disposable circuit comprises a blood withdrawal line 6 connected to an inlet of the primary chamber 3, and a blood return line 7 connected to an outlet of the primary chamber 3. In use, the blood withdrawal line 6 and the blood return line 7 are connected to a vascular access device in communication with the patient vascular system. For example the blood withdrawal and return lines may be connected to a fixed catheter (e.g., a central venous catheter for acute patients) or to respective needles, in particular albeit in a non-limiting extent metal needles (to be inserted in e.g., an arteriovenous fistula for chronic patients), or other access device (not shown in annexed figures) which, in a first configuration of the disposable circuit herein described as "operative configuration" or "treatment configuration", is placed in fluid communication with the patient vascular system, such that blood may be withdrawn through the blood withdrawal line, flowed through the primary chamber and then returned to the patient's vascular system through the blood return line. The blood return line 7 and the blood withdrawal line 6 are made of flexible plastic material. In a non-limiting solution, the blood withdrawal line 6 and the blood return line 7 are made of transparent plastic material, allowing to identify presence of blood.

In more detail, the blood withdrawal line 6 comprises a first end connected to the inlet of the primary chamber 3 and a second end opposite to the first end connected to an arterial connector 40, which is the last part of the disposable circuit an placed downstream the arterial access 40a providing access to the vascular system of the patient "P"; the blood return line 7 comprises a first end connected to the outlet of the primary chamber 3 and a second end opposite to the first end connected to a venous connector 41, which is the last part of the disposable circuit upstream the venous access 41a of the patient "P".

In the course of the present disclosure reference will made to "downstream" and "upstream" for indicating the position of elements with respect to others. As marked in figure 1, with arrow F, the normal flow of fluid imposed by the blood pump 11 is from the arterial connector 40 to the venous connector 41; thus wording "downstream" and "upstream" will be referred considering such flow. Thus in the operative configuration the fluid moves from the arterial connector 40 through the portion 6a of the blood withdrawal line 6 up to the blood pump 11 that forces the flow at the inlet of the primary chamber 3 and then through the filter 2 up to the outlet of the primary chamber 3. The fluid then moves into the blood return line 7 up to its second end connected to the venous connector 41. For instance, the blood pump 11 lies upstream the primary chamber 3, and the outlet of the primary chamber 3 is downstream the inlet of the primary chamber 3 and/or the blood pump 11.

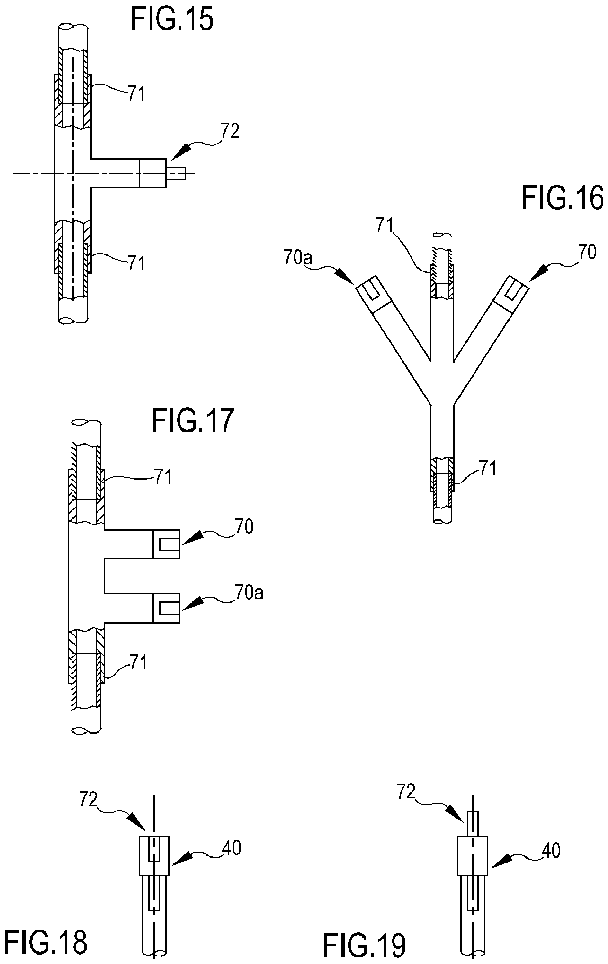

The arterial connector 40 and the venous connector 41 are non-disconnectable connectors, which are provided with a port fixed (e.g., welded or glued) to a respective tube portion (the blood withdrawal line and the blood return line), and a free port which is detachably connectable to the respective arterial access 40a or venous access 41a (for example the arterial and venous needles or the catheter). The free port of the arterial and venous connector may be of a male type or of a female type. Preferably, the free ports of both the arterial and venous connectors 40, 41 are of a same type. Figure 18 and 19 show non-limiting and schematic embodiments of arterial or venous connectors whose free port 72 is of a female type (figure 18) or of a male type (figure 19). Luer connectors are preferably used. Being of the same connection type, the arterial connector 40 and the venous connector 41 cannot be directly connected together.

At least one air separator, such as a bubble trap 8, is present in the blood circuit 6, 7, and preferably on the blood return line (a further bubble trap may be provided in the withdrawal line, too); moreover, a safety clamp 9 of the apparatus is active on the blood return line, and it is usually placed downstream the bubble trap 8 and close to the venous connector 41. The safety clamp 9 may be controlled by a control unit 10 of the apparatus 1. It shall be noted that albeit an air separator and a safety clamp are optional elements, their presence is preferable.

Additionally, the apparatus further comprises a bubble sensor 8a, active in correspondence of a portion of the return line 7 between bubble trap 8 and safety clamp 9; the bubble sensor is connected to the control unit 10 and sends to the control unit signals in case one or more bubbles are detected; in such a case, the control unit may stop the treatment and cause closure of the clamp 9. A corresponding safety clamp of the apparatus (not represented) may be provided in correspondence of the blood withdrawal line 6. The safety clamp is active on the blood withdrawal line 6 immediately downstream the arterial connector 40.

As shown in figure 1, the blood flow through the blood lines is controlled by a blood pump 11, for instance a peristaltic blood pump, acting either on the blood withdrawal line or on the blood return line. The blood pump 11 is a peristaltic blood pump, part of the apparatus 1. An operator may enter a set value for the blood flow rate Q_{B} through a user interface 12 (or the apparatus may calculate the blood flow rate based on other set parameters) and the control unit 10, during treatment, is configured to control the blood pump based on the set/calculated blood flow rate. In the course of the present description it is indicated that the control unit is "configured" or "programmed" to execute certain steps: this may be achieved in practice by any means which allow configuring or programming the control unit.

The apparatus 1 is provided with a (graphic) user interface which is configured to allow setting parameters for the treatment and to impart commands and controls to the apparatus 1 in the various operative configurations thereof. The user interface may comprise a screen or monitor, optionally touch sensitive, and one or more switches and/or pushbuttons specifically configured to cause a particular operation of the apparatus 1.

The disposable circuit further comprise an effluent fluid line 13 connected, at one end, to an outlet of the secondary chamber 4 and, at the other end, to an effluent fluid container 14 collecting spent fluid extracted from the secondary chamber. Alternatively the effluent line 13 may direct spent fluid directly to a drain. The effluent fluid line is configured to be interfaced with an effluent fluid pump 17 of the apparatus: the effluent fluid pump 17 operates on the effluent fluid line under the control of the control unit 10 to regulate the flow rate Q_{eff} across the effluent fluid line.

A dialysis fluid line 19 is connected at one end with a dialysis fluid container 20 (or to a dialysis fluid preparation device) and at its other end with the inlet of the secondary chamber 4 of the filtration unit. The dialysis fluid line 19 of the fluid circuit is configured to cooperate with a dialysis liquid pump 17a of the apparatus 1. The dialysis liquid pump 17a works on the dialysis liquid fluid line under the control of said control unit 10, to supply fresh dialysis fluid from the dialysis liquid container to the secondary chamber at a flow rate Q_{dial}.

According to certain embodiments for acute treatment, disposable circuits may comprise the above mentioned effluent fluid line 13 and dialysis fluid line 19 as disposable components integrally fixed to the filtration unit. At the end of the treatment, the entire disposable set is disposed. The dialysis fluid line 19 received fresh fluid from container 20, the spent dialysis fluid is routed to the effluent bag 14.

Differently, chronic renal treatment disposables include neither the dialysis fluid line nor the effluent fluid line as disposable parts. The dialysis fluid line and the effluent fluid line are non-disposable fluid paths arranged into the apparatus 1. A detachable connection is provided to removably place in fluid communication the dialysis fluid line and the effluent fluid line respectively to the inlet and the outlet of the secondary chamber 4 of the filtration unit; the latter being part of the disposable circuit. Dialysis fluid is either on-line prepared mixing water with concentrates, or received from a central dialysis fluid preparation plant.

In the context of the present disclosure, wording "upstream" and "downstream" will be referred in accordance to the fluid flow imposed by the effluent fluid pump 17 and by the dialysis pump 17a, which in figure 1 is marked by the arrow F'. Effluent fluid pump 17 and dialysis pump 17a cooperate to force fluid flow from the dialysis fluid container 20 through the dialysis fluid line 19, to the dialysis pump 17a, then to the inlet of the secondary chamber 4 through the filter 2 up to the outlet of the secondary chamber 4, when fluid enters the effluent fluid line 13 to flow at the effluent fluid pump 17 and then to the effluent fluid container 14.

The dialysis fluid pump 17a and the effluent fluid pump 17 are part of means for regulating the flow of fluid through the respective lines and, as mentioned, are operatively connected to the control unit 10 which controls the pumps. The control unit 10 is also connected to the user interface 12, for instance a graphic user interface, which receives operator's inputs and displays the apparatus outputs. For instance, the graphic user interface 12 may include a touch screen, a display screen and hard or soft keys for entering user's inputs or a combination thereof.

The disposable circuit shown in figure 1 may include an infusion line 21 connected, at one end, with a portion 6a of the blood withdrawal line 6 positioned upstream the blood pump 11 and, at its other end, with an infusion fluid container 23, which for instance may contain a regional anticoagulant such as a citrate solution, or a replacement solution or other fluids. This infusion line is hereinafter referred to as pre-blood pump pre-dilution infusion line 21. The means for regulating the fluid flow comprises a pump 22, for instance a peristaltic pump controlled by control unit 10, acting on a segment of the pre-blood pump pre-dilution infusion line 21 to regulate a pre-blood pump infusion rate Q_{pbp}. The pump 22 is part of the apparatus 1.

For the purposes of the present disclosure the three way connector above described is a "non-disconnectable connector", that means that in the normal use the connector is not configured to be disconnected from any portion of conduit connected at any of its three ports or, equivalently, that the portion of conduits connected at any of its three ports are fixedly jointed thereto, e.g. through bonding or sealing.

The embodiment of figure 1 also includes a post-blood pump pre-dilution fluid line 15 connected, at one end, directly with a portion 6a of the blood withdrawal line 6 positioned downstream the blood pump 11 and, at its other end, with a further infusion fluid container: this post-blood pump pre-dilution fluid line 15 supplies replacement fluid from an infusion fluid container 16 connected at one end of the post-blood pump pre-dilution fluid line 15. The apparatus further comprises an infusion pump 18, for instance a peristaltic pump, operating on the post-blood pump pre-dilution fluid line 15 to regulate the flow rate Qₚᵣₑ through the line 15.

When referring to the post blood pump pre-dilution infusion line 15, or to the pre-blood pump pre-dilution infusion line 21, wording "upstream" and "downstream" will be referred to the normal fluid flow during the operative configuration. Fluid flows from the container 16, 23, through the portion of conduit line on which the pump 18, 22 of any of the infusion lines acts, up to the non-disconnectable connector which connects the infusion line with the blood withdrawal line 6.

Alternatively to, or in combination with the pre-dilution fluid line 15, the apparatus of figure 1, may also present a post-dilution fluid line 25 connected at one end with a further container 26 of infusion liquid and connected at its other end with the blood return line 7. A further pump 27, for instance a peristaltic pump, may act under the control of control unit 10 on the post-dilution fluid line 25 and thus also be part of said means for regulating the flow through the fluid lines.

When referring to the post-dilution fluid line 25, wording "upstream" and "downstream" will be referred to the normal fluid flow during the operative configuration. Fluid flows from the container 26, through the portion of conduit line on which the pump 27 acts, up to the non-disconnectable connector which connects the infusion line with the blood return line 7.

Alternatively or in combination with the aforementioned fluid lines, the apparatus of figure 1 may comprise one or more auxiliary lines, which are connected, at one end, with the blood circuit, for example to the blood withdrawal line 6. Figure 1 shows a single auxiliary line 42. A further pump, not shown in figure 1, for instance a peristaltic pump, may act under the control of control circuit 10 on the auxiliary line and thus also be part of said means for regulating the flow through the fluid lines.

When referring to the any of the auxiliary lines, wording "upstream" and "downstream" will be referred to the normal fluid flow which flows from the free end of the auxiliary line to the end which is connected to the blood circuit 6, 7.

The apparatus 1 may also comprise a first scale 33 operative for providing weight information relative to the amount of the spent fluid collected in the effluent fluid container 14; a second scale 34 operative for providing weight information relative to the amount of the fluid supplied from the infusion fluid container 16 via a post blood pump pre-dilution infusion line 15; a third scale 35 operative for providing weight information relative to the amount of the fluid supplied from dialysis fluid container 20. In case more infusion lines would be present, as a pre-blood pump pre-dilution infusion line 21, post-dilution fluid line 25, then a respective scale 36 and 37 could be present to provide weight information relative to the amount of fluid supplied from infusion containers 23, 26. The scales are all connected to the control unit 10 and provide said weight information for the control unit 10 to determine the actual quantity of fluid in each container and/or the actual flow rate of fluid supplied by or received in each container. The control unit 10 may then be configured to receive treatment selection information and check if the user entered the required dialysis parameters. The control unit may also be configured to receive weight information from the first scale and, depending upon the selected treatment, from the scales associated to the container supplying the lines involved in the delivery of the selected treatment) and to control, at least at the beginning of the treatment, the flow rate of at least one of the effluent fluid, the infusion fluid, the dialysis fluid by controlling said means for regulating based on said weight information, and the initial set values.

From a structural point of view one or more, optionally all the containers 14, 16, 20, 23, 26 may be disposable plastic containers, preferably soft plastic bags which are hang on a support carried by the respective scale. The means for regulating flows typically comprises pumps, although other regulating means as valves or combinations of valves and pumps could be used. The scales may comprise piezoelectric sensors, or strain gauges, or spring sensors, or any other type of transducer able to sense forces applied thereon. Although the examples in the figures show use of scales for determining the amount of fluid in the respective containers and for allowing calculation of the respective flow rates through the various lines, it should be noted that the above described aspects of the invention are compatible also with blood treatment machines using volumetric sensors for determining flow rates or combinations of mass and volumetric sensors.

Though the previous description mainly referred to dialysis apparatus, embodiments of the present disclosure further encompasses hemoperfusion. Hemoperfusion is a method of filtering the blood extracorporeally to remove one or more toxins. As with other extracorporeal methods, the blood travels from the patient into a blood withdrawal line 6, gets filtered in a filtration unit 2, and then travels back into the patient through a blood return line 7, typically by using venovenous access. In hemoperfusion, the blood perfuses a filter composed of artificial cells filled with activated carbon or another microporous material. In an hemoperfusion apparatus, no infusion line may be present. In such a case, the first and second auxiliary connectors are placed on the blood line, for examples in the positions as below disclosed according to the fourth embodiment. As a possible further hemoperfusion configuration, a pre-blood pump pre-infusion line may be included for injecting a solution into the blood upstream the blood pump. In this latter embodiment, the first and the second auxiliary connectors may be placed on the pre-blood pump pre-infusion line, or may remain on the blood circuit (or one connector may be placed on the pre-blood pump pre-infusion line and the other one may be placed on the blood line.

Further, the apparatus of embodiments may be (alternatively or in addition) an extracorporeal CO₂ Removal (ECCO2R) device; the blood line circuit may be connected to a CO₂ removal filtration unit 2 and the apparatus may or may not include any infusion lines. The first and second auxiliary connectors may be placed on the blood circuit in case no infusion line is available, or on either the blood circuit or the infusion line, if present.

The disposable circuit of the present disclosure is designed to allow for temporary disconnection and then patient reconnection to the same disposable circuit to continue a (temporary) interrupted treatment. To achieve the task, the disposable set is reconfigurable for allowing fluid (e.g., blood) recirculation mainly in the blood circuit. Alternatively to the treatment (operative) configuration above described, another configuration for the disposable circuit herewith described is here referred as "recirculation configuration". In said configuration, the patient is disconnected from the disposable circuit and fluid therein present may be recirculated for allowing the patient to move away from the extracorporeal blood treatment apparatus. Thanks to this aspect, a patient may be temporarily disconnected from the apparatus 1 to perform or undergo other exams or treatments while the treatment performed with the apparatus 1 is suspended in a very easy way, without requesting expensive complete change of disposable circuit and with a reduced health risk in relation to the disconnection - recirculation - reconnection operations. Further, no additional components are required to achieve temporary disconnection, such as additional 'Y' connector, saline bag, etc. An easy switching or reconfiguration capability between the operative configuration and the recirculation configuration further eases the activity of the qualified operators that help the patient throughout the performance of the treatment. During the recirculation configuration, i.e. after the control unit 10 has been properly set for the recirculation configuration, the target means values of flow rate(s) and/or prescribed does is interrupted, average values that may have been calculated and/or measured by the control unit 10 or any of the sensors operatively connected thereto are preferably stored in a memory. Calculation and/or measurement thereof, starts back at the end of the temporary disconnection of the patient, when the control unit 10 is properly set for re-establishing the original operative configuration of blood treatment; optionally, albeit preferably, the control unit 10 may be configured to allow calculation and/or measurement of partial values before the instauration of the recirculation configuration and after the re-instauration of the operative configuration.

For this purpose, several embodiments of the disposable circuit have been conceived as being provided with a first and a second auxiliary connector, which in the recirculation configuration are connected one with the venous connector 40 and the other with the arterial connector 41 for the purposes of defining a properly closed circuit allowing the fluid recirculation under the action of the blood pump 11. Albeit in the detailed disclosure of the embodiments always two auxiliary connectors are disclosed, in line of principle at least one auxiliary connector could be sufficient to make the closed circuit. Indeed, though not preferred, it is possible to connect one blood line end (venous or arterial connector) to the first auxiliary connector and to leave the other blood line end as a dead end (the second blood end, namely the dead end, is simply clamped). The fluid inside the closed loop may then be recirculated. Preferably, saline or other replacement fluid should be recirculated (i.e., not blood).

Preferably, but in a non-limiting extent, the auxiliary connectors are three way connectors having a main inlet and a main outlet receiving pieces of the main line where they are placed and are provided with at least one disconnectable port which may be sealed through a cap or, alternatively, which may be of a self-sealing type. This latter type is such that the port automatically seals when another connector is disconnected from the disconnectable port of the auxiliary connector and automatically opens when the other connector is connected to the disconnectable port of the auxiliary connector. A self-sealing type auxiliary connector helps preventing unwanted fluid (in particular blood) leakage when the patient treatment is interrupted and the configuration of the disposable circuit is switched between the operative configuration and the recirculation configuration, reduces the risk of inclusion of air bubbles in the blood circuit and further eases the operations performed by the qualified operators that assist the patient.

The auxiliary connector may have a wide variety of shapes, and may be provided with at least one first, or even with a second, disconnectable and free port which may be used to connect the auxiliary connector with another auxiliary connector and/or with the other one of the venous and the arterial connector. Non-limiting configurations of the connectors according to the present disclosure comprise three way auxiliary connectors like a "T" shaped connector (figure 15).

The illustrated connector (being it the first or the second auxiliary connector) comprises a first and a second non-disconnectable service port 71 fixed (e.g., sealed) to respective flexible tube portions. For example, the tube portions may be respective tube portions of the pre-blood pump pre-dilution line 21; alternatively, the tube portions may be respective tube portions of any other infusion line or tube portions of the blood withdrawal or return line. The disconnectable port 72 represented only schematically may be a male (or female) Luer connector configured to couple with the corresponding female (or male) Luer connector 40, 41 of the blood withdrawal line 6 or of the blood return line 7. Obviously a "Y" shaped connector, a half-snowflake shaped connector or another connector of different geometry may be used.

Looking at figure 16, the first and second auxiliary connectors may be made in a single piece, wherein the first and the second non-disconnectable service port 71 are still fixed (e.g., sealed) to respective flexible tube portions, but the merged connector includes two disconnectable ports 70, 70a for receiving the withdrawal line connector 40 and the return line connector 41, respectively. Also in this design, if the withdrawal line connector 40 and the return line connector 41 are Luer male connectors, then the two disconnectable ports 70 are Luer female connectors and vice versa.

The geometry of the 'integral' connector may also be different, such as the embodiment of figure 17, wherein the two disconnectable ports 70 emerge parallel to each other on the same lateral side of the device.

Figure 1 presents, in a single schematic diagram, principal and non-limiting embodiments conceived by the inventors that are further herewith described in detail.

### First embodiment

In a first embodiment the first auxiliary connector 53 and the second auxiliary connector 52 are arranged on the pre-blood pump pre-dilution infusion line 21, in a portion of conduit that once the disposable circuit is properly installed on the apparatus 1, lies downstream the pump 22 arranged on the pre-blood pump pre-dilution infusion line 21. The first auxiliary connector 53 is arranged downstream the second auxiliary connector 52.

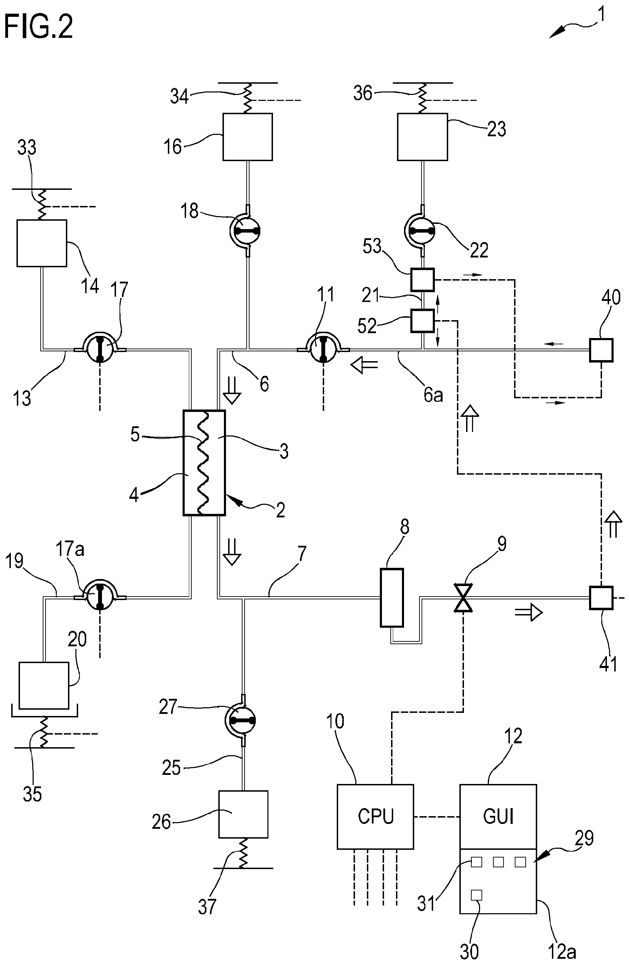

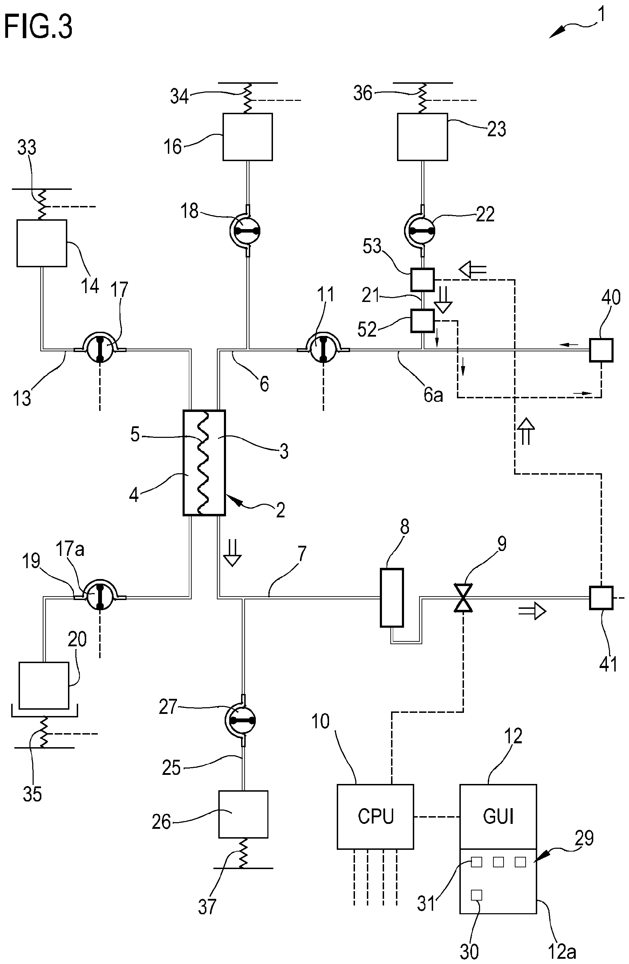

In the recirculation configuration, alternatively:
- the first auxiliary connector 53 is connected to the arterial connector 40 and the second auxiliary connector 52 is connected to the venous connector 41 (figure 2); or
- the first auxiliary connector 53 is connected to venous connector 41 and the second auxiliary connector 52 is connected to the arterial connector 40 (figure 3).

In the recirculation configuration of figure 2, full flow rate (double arrow) is present in the blood withdrawal line 6 downstream the pre-blood pump pre-dilution infusion line 21 and in the blood return line 7; a full flow rate arrives to the second auxiliary connector 52 and there the flow rate is split into partial flow rates (single arrows) that are present in the portions of lines connecting the second auxiliary connector 52 to the first auxiliary connector 53 and then in the portion of line that connects the arterial connector 40 to the pre-blood pump pre-dilution infusion line 21 injection point in the blood withdrawal line 6. Here the two partial flow rates merges again generating a full flow rate.

In figures 2 and 3, a double arrow indicates a full flow rate and its direction along the lines; a single arrow indicates a split (partial) flow rate and its direction along the line portion. The same representation is used in respect to all the figures showing recirculation.

In the first embodiment the first and the second auxiliary connectors are both three-way connectors which are provided each with a disconnectable port 70 configured to be connected to the disconnectable port 72 of one connector selected between the venous connector 40 and the arterial connector 41 in accordance to one of the two possible alternatives above identified. The disconnectable port 72 of the first and second auxiliary connectors 53, 52 are of a type which opposite to the type of the disconnectable port of the venous and arterial connectors. In other words, when the disconnectable port 72 of the first and second auxiliary connectors 53, 52 are of a male type, the disconnectable port of the arterial and venous connector is of a female type, and vice-versa.

### Second embodiment

In a second embodiment the first auxiliary connector 51 and the second auxiliary connector 50 are arranged on the post-blood pump pre-dilution infusion line 15, in a portion of conduit that once the disposable circuit is properly installed on the apparatus 1, lies downstream the pump 18 arranged on the post-blood pump pre-dilution infusion line 15. The first auxiliary connector 51 is arranged downstream the second auxiliary connector 50.

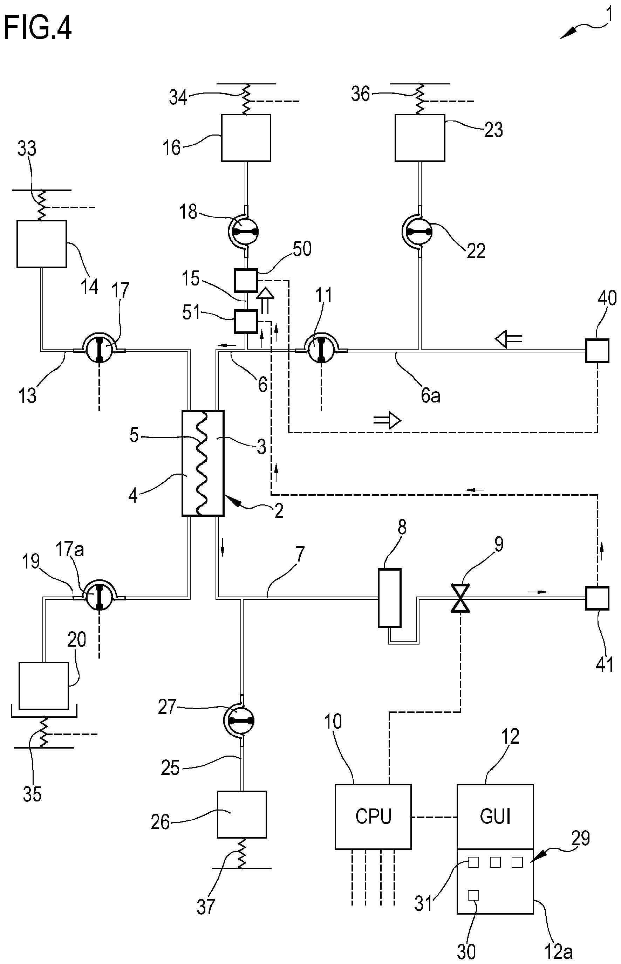

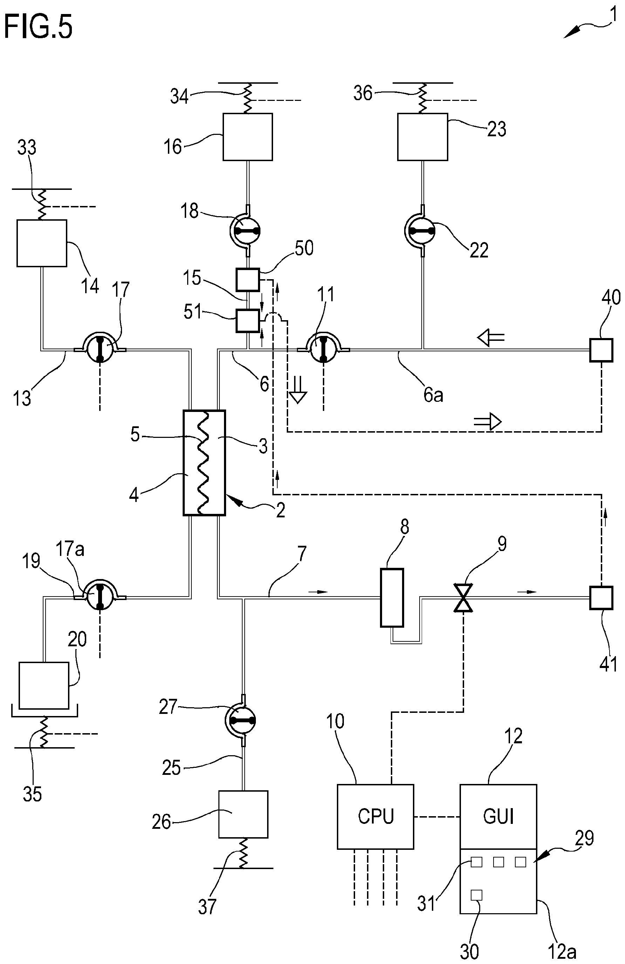

In the recirculation configuration, alternatively:
- the first auxiliary connector 51 is connected to venous connector 41 and the second auxiliary connector 50 is connected to the arterial connector 40 (figure 4); or
- the first auxiliary connector 51 is connected to the arterial connector 40 and the second auxiliary connector 50 is connected to the venous connector 41 (figure 5).

In the recirculation configuration of figure 4, full flow rate is present in the blood withdrawal line 6 upstream the post-blood pump pre-dilution infusion line 15 and, the full flow rate is split at the injection point of line 15 and partial flow rate is present in the blood return line 7, in the venous connector 41 up to the first auxiliary connector 51; here the partial flow coming up from the blood withdrawal line and the partial flow at the to the first auxiliary connector 50 merge and in the portion of line that connects the first auxiliary connector 51 to the second auxiliary connector 50 and up to the blood withdrawal line 6 there is full flow, as represented.

In the second embodiment the first and the second auxiliary connectors 50, 51 are both three-way connectors which are provided each with a disconnectable port 70 configured to be connected to another disconnectable port 72 of one connector selected between the venous connector 40, and the arterial connector 41 in accordance to one of the two possible alternatives above identified. The disconnectable port 72 of the first and second auxiliary connectors 51, 50 are of a type which opposite to the type of the disconnectable port of the venous and arterial connectors. In other words, when the disconnectable port 72 of the first and second auxiliary connectors 51, 50 are of a male type, the disconnectable port of the arterial and venous connector is of a female type, and vice-versa.

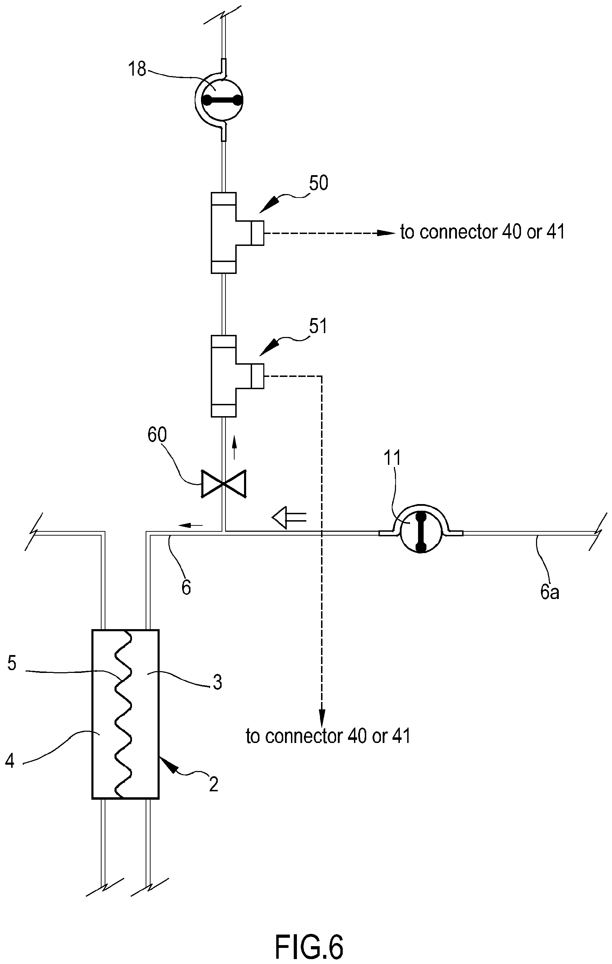

As schematically disclosed in figure 6, optionally, the post-blood pump pre-dilution infusion line 15 may comprise a pressure drop valve or flow resistor 60, which is in particular and in a non-limiting extent installed downstream the first auxiliary connector 51, just upstream the non-disconnectable three way connector that connects the post-blood pump pre-dilution infusion line 15 to the blood withdrawal line 6. The pressure drop valve or flow resistor 60 is configured to even flow resistances during recirculation and may be detachable or fixedly installed on the fluid line; optionally, the drop valve or flow resistor may be of a variable-resistance type. The aim of the drop valve or flow resistor 60 is to make the resistances of the respective portions of the pipe similar so that the flows generated in each of the circuit branches are as similar as possible.

### Third embodiment

In a third embodiment the first auxiliary connector 57 and the second auxiliary connector 56 are arranged on the post-dilution fluid line 25, in a portion of conduit that once the disposable circuit is properly installed on the apparatus 1, lies downstream the pump 27 arranged on the post-dilution fluid line 25. The first auxiliary connector 57 is arranged downstream the second auxiliary connector 56.

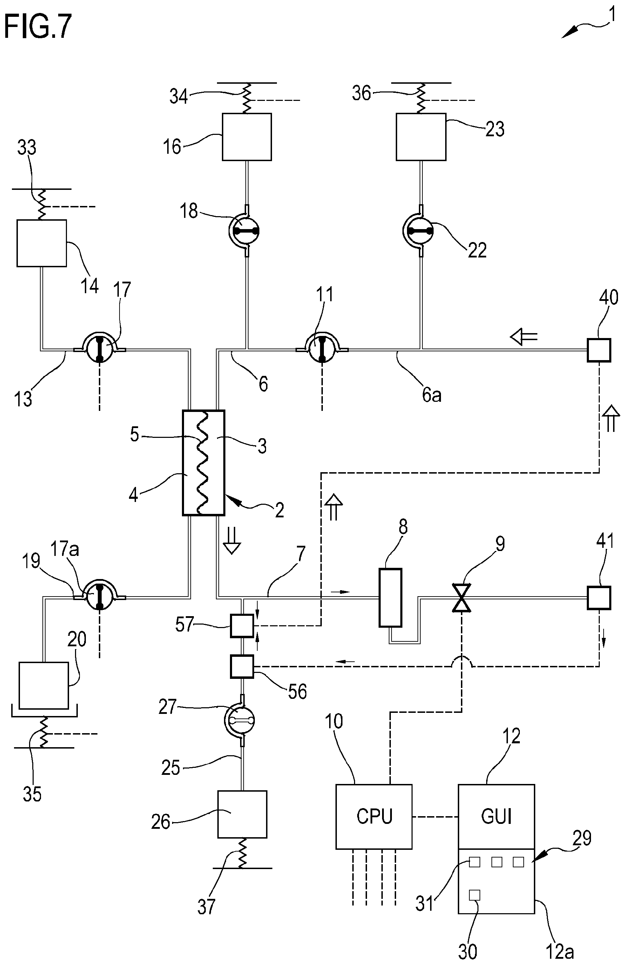

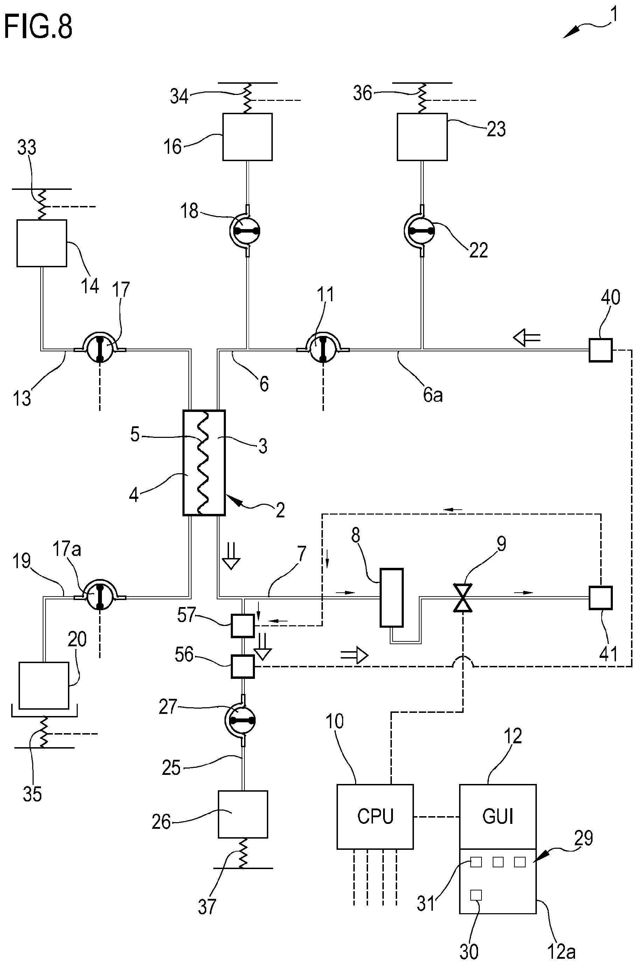

In the recirculation configuration, alternatively:
- the first auxiliary connector 57 is connected to the arterial connector 40 and the second auxiliary connector 56 is connected to the venous connector 41 (figure 7); or
- the first auxiliary connector 57 is connected to venous connector 41 and the second auxiliary connector 56 is connected to the arterial connector 40 (figure 8).

In the recirculation configuration of figure 7, full flow rate is present in the blood withdrawal line 6 and in the portion of blood return line 7 that departs from the outlet of the primary chamber 3 and arrives to the non-disconnectable connector which connects the blood return line 7 to the post-dilution fluid line 25; partial flow rate is present in the portions of lines connecting the first auxiliary connector 57 to the second auxiliary connector 56 and then in the portion of the blood return line 7 downstream the post-dilution fluid line 25.

In the third embodiment the first and the second auxiliary connectors are both three-way connectors which are provided each with a disconnectable port 70 configured to be connected to another disconnectable port 72 of one connector selected between the venous connector 40, the arterial connector 41 in accordance to one of the two possible alternatives above identified. The disconnectable port 72 of the first and second auxiliary connectors 57, 56 are of a type which opposite to the type of the disconnectable port of the venous and arterial connectors. In other words, when the disconnectable port 72 of the first and second auxiliary connectors 57, 56 are of a male type, the disconnectable port of the arterial and venous connector is of a female type, and vice-versa.

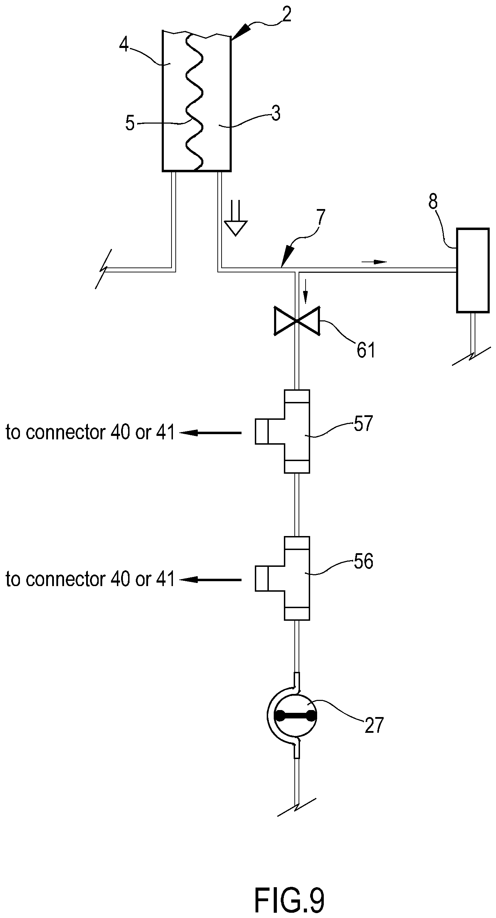

As schematically disclosed in figure 9, optionally, the post-dilution fluid line 25 may comprise a pressure drop valve or flow resistor 60, which is in particular installed downstream the first auxiliary connector 57 just upstream the non-disconnectable three way connector that connects the post-dilution fluid line 25 to the blood return line 7. The pressure drop valve or flow resistor 60 is configured to even flow resistances during recirculation and may be detachable or fixedly installed on the fluid line; optionally, the drop valve or flow resistor may be of a variable-resistance type..

### Fourth embodiment

In a fourth embodiment the first auxiliary connector 59 and the second auxiliary connector 58 are arranged both on the blood circuit, being configured so that the first auxiliary connector 59 is arranged on the blood return line 7 while the second auxiliary connector 58 is arranged on the blood withdrawal line 6. Preferably, albeit in a non-limiting extent, the second auxiliary connector 58 lies on the portion 6a of the blood withdrawal line 6 that once the disposable circuit is properly installed on the apparatus 1, lies upstream the blood pump 11; the first auxiliary connector 59 is preferably installed upstream the bubble detector 8a and the safety clamp 9. Therefore, the first auxiliary connector 59 is arranged downstream the second auxiliary connector 58.

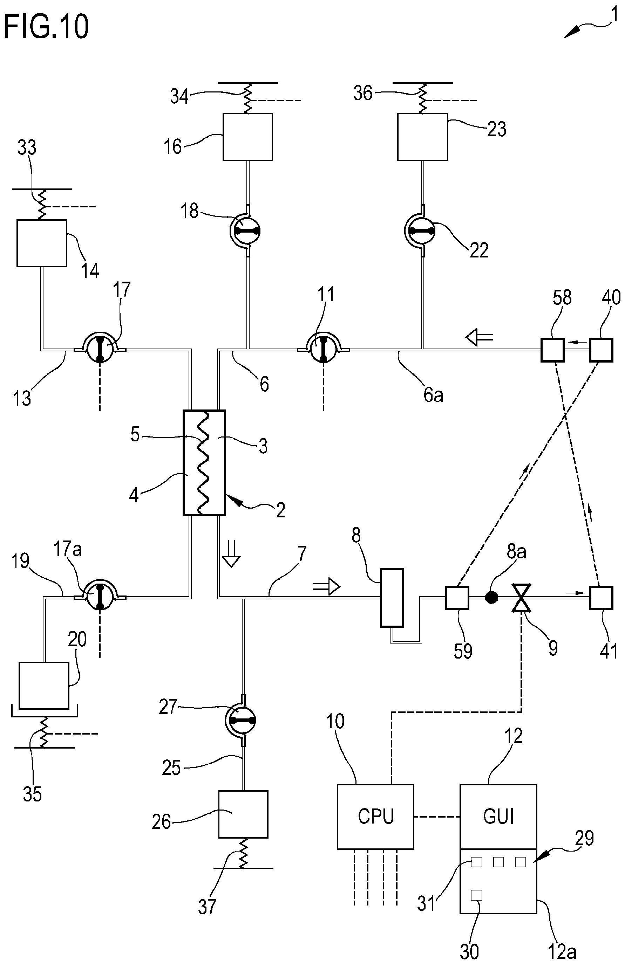

In the recirculation configuration of figure 10, the first auxiliary connector 59 is connected to the arterial connector 40 and the second auxiliary connector 58 is connected to the venous connector.

In the recirculation configuration of figure 10, full flow rate is present in the blood withdrawal line 6 downstream the second auxiliary connector 58 and upstream the first auxiliary connector 59, i.e. in the respective portions of blood withdrawal lines 6 between the second auxiliary connector 58 and the inlet of the primary chamber 3, and in the portion of blood return line 7 between the outlet of the primary chamber and the first auxiliary connector 59; partial flow rate is present in the portions of blood withdrawal line 6 that lies between the second auxiliary connector 58 and the arterial connector 40 and in the blood return line 7 that lies between the first auxiliary connector 59 and the venous connector 41.

In the fourth embodiment the first and the second auxiliary connectors are both three-way connectors which are provided each with a disconnectable port 70 configured to be connected to another disconnectable port 72 of one connector selected between the venous connector 40, the arterial connector 41. The disconnectable port 72 of the first and second auxiliary connectors 59, 58 are of a type which opposite to the type of the disconnectable port of the venous and arterial connectors. In other words, when the disconnectable port 72 of the first and second auxiliary connectors 59, 58 are of a male type, the disconnectable port of the arterial and venous connector is of a female type, and vice-versa.

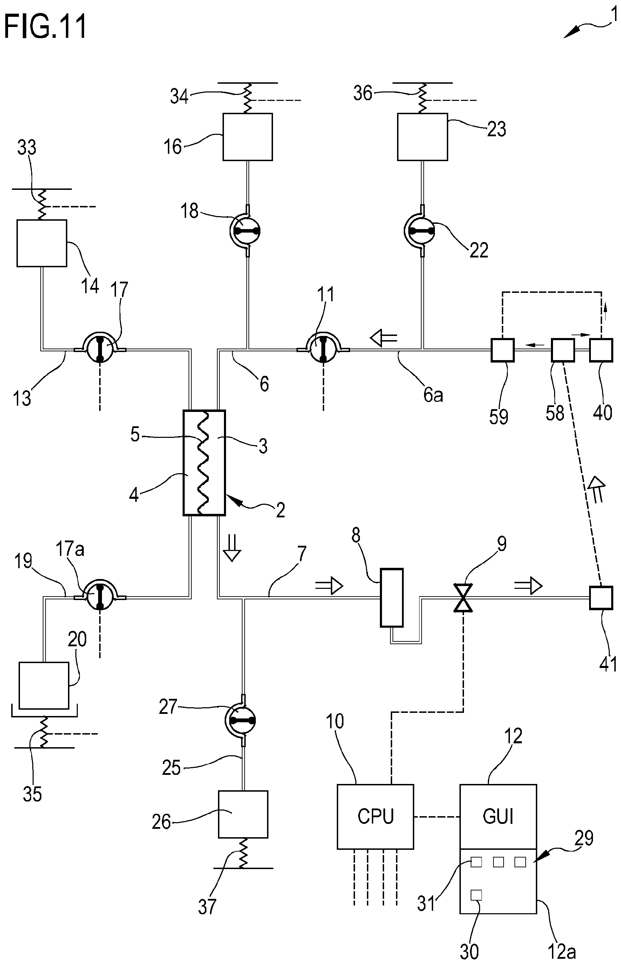

Figure 11 shows an alternative connection wherein the first and the second auxiliary connectors are both placed on the blood withdrawal line 6, in the line portion 6a upstream the blood pump 11; the venous connector 41 is connected directly to the second auxiliary connector 58; the arterial connector 40 is connected directly to the first auxiliary connector 59 which is placed downstream the second auxiliary connector 58. It may be noted that this latter configuration is not to be considered as preferred but anyway still allowable to define a closed circuit allowing fluid recirculation as intended in the present disclosure.

### Fifth embodiment

In a fifth embodiment the first auxiliary connector 55 and the second auxiliary connector 54 are arranged both on a single auxiliary line which is installed in a portion of e.g., the blood withdrawal line 6, preferably but in a non-limiting extent, in a portion of the blood withdrawal line that once the disposable circuit is properly arranged on the apparatus 1 lies upstream the blood pump 11. In the fifth embodiment, the first auxiliary connector 55 is arranged downstream the second auxiliary connector 54.

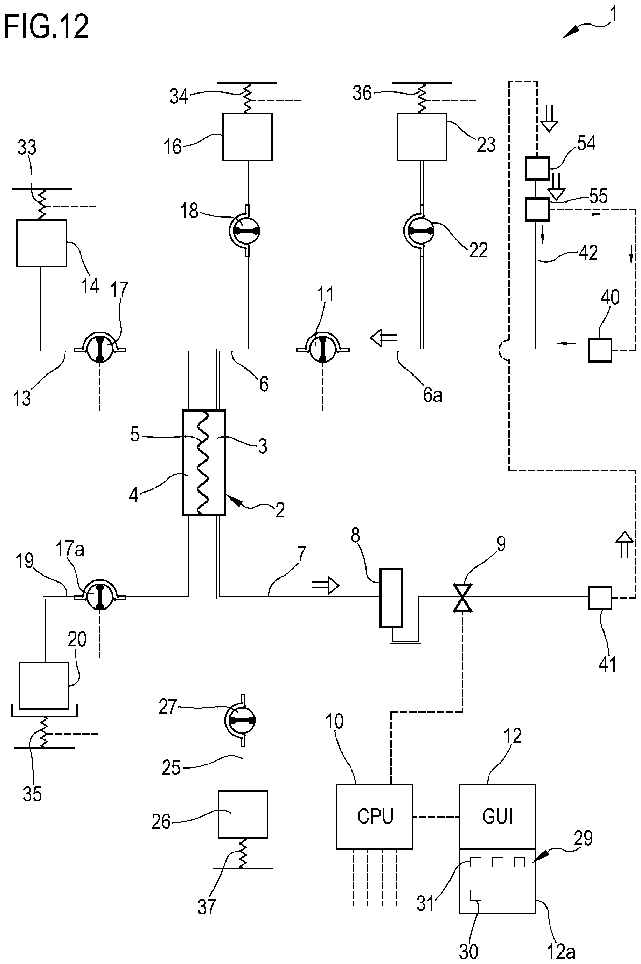

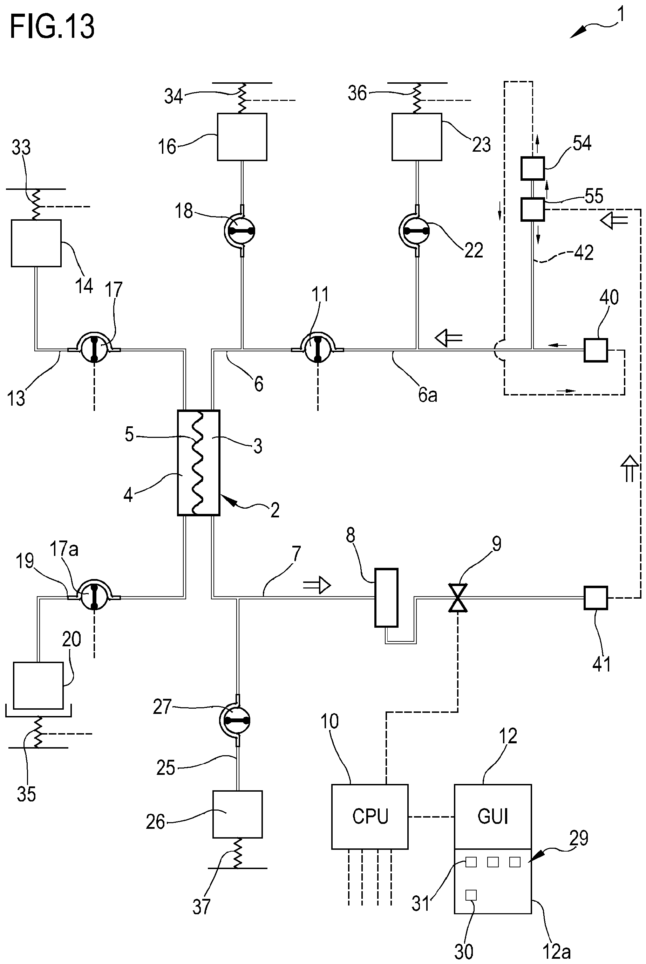

In the recirculation configuration, alternatively:
- the first auxiliary connector 55 is connected to the arterial connector 40 and the second auxiliary connector 54 is connected to the venous connector 41 (figure 12); or
- the first auxiliary connector 55 is connected to venous connector 41 and the second auxiliary connector 56 is connected to the arterial connector 40 (figure 13).

In the recirculation configuration of figure 12, full flow rate is present in the blood withdrawal line 6 downstream the auxiliary line 42 and in the blood return line 7; partial flow rate is present in the auxiliary line 42 and in the portion of blood withdrawal line 6 which is comprised between the three way non-disconnectable connector which connects one end of the auxiliary line 42 with the blood withdrawal line 6 and the second end of the blood withdrawal line 6 which ends on the arterial connector 40.

In the fifth embodiment one connector between the first and the second auxiliary connectors is a three-way connector which is provided with a disconnectable port 70 configured to be connected to another disconnectable port 72 of one connector selected between the venous connector 40, the arterial connector 41 in accordance to one of the two possible alternatives above identified. The other connector is a two way connector provided with a disconnectable port and a non-disconnectable port fixedly installed on a respective tube portion of the auxiliary line 42. The disconnectable port 72 of the first and second auxiliary connectors 55, 54 are of a type which opposite to the type of the disconnectable port of the venous and arterial connectors. In other words, when the disconnectable port 72 of the first and second auxiliary connectors 55, 54 are of a male type, the disconnectable port of the arterial and venous connector is of a female type, and vice-versa.

The fifth embodiment is similar to the first embodiment; however, a service line 42 instead of a treatment line 21 is used for the temporary disconnection and recirculation.

### Sixth embodiment

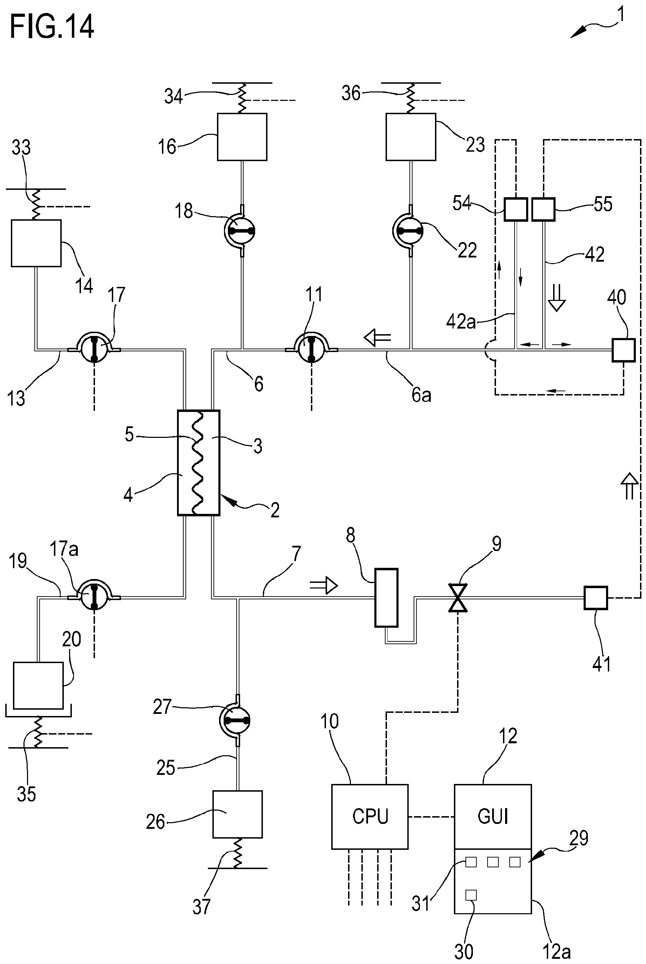

A sixth embodiment of the disposable circuit is schematically shown in figure 14; it is provided with a first auxiliary line 42 and a second auxiliary line 42a arranged on a portion of the blood withdrawal line 6 which - preferably albeit in a non-limiting extent - when the disposable circuit is properly arranged on the apparatus 1 lie both upstream the blood pump 11 and optionally upstream the pre-blood pump pre-infusion line 21. Each of the first and second auxiliary lines 42, 42a are provided with a first end connected to the blood withdrawal line 6 and a second end which terminates on a respective first auxiliary connector 55 arranged on the first auxiliary line 42 and a second auxiliary connector 54 arranged on the second auxiliary line 42a.

In the recirculation configuration, alternatively:
- the first auxiliary connector 55 is connected to the venous connector 41 and the second auxiliary connector 54 is connected to the arterial connector 40 (figure 15); or
- the first auxiliary connector 55 is connected to arterial connector 40 and the second auxiliary connector 56 is connected to the venous connector 41 (not depicted in any figure, since substantially symmetrical to the configuration of figure 15).

In the recirculation configuration of figure 15, full flow rate is present in the blood withdrawal line 6 downstream the second auxiliary line 42a and in the blood return line 7; partial flow rate is present in the first, second auxiliary lines 42, 42a and in the portion of blood withdrawal line 6 which is comprised between the three way non-disconnectable connector which connects one end of the first auxiliary line 42 with the three way non-disconnectable connector which connects one end of the second auxiliary line 42a upstream up to the second end where the blood withdrawal line 6 ends with the arterial connector 40.

In the sixth embodiment both the first and the second auxiliary connectors are two way connectors provided with a disconnectable port and a non-disconnectable port fixedly installed on a respective tube portion of the respective first, second auxiliary line 42. The disconnectable port 72 of the first and second auxiliary connectors 55, 54 are of a type which opposite to the type of the disconnectable port of the venous and arterial connectors. In other words, when the disconnectable port 72 of the first and second auxiliary connectors 55, 54 are of a male type, the disconnectable port of the arterial and venous connector is of a female type, and vice-versa.

Finally, it is not excluded that the first and second auxiliary connectors may be placed one on any of the described infusion lines and the other on the blood circuit, properly allowing for blood recirculation when connected to the arterial and venous connectors 40, 41.

### Method of operation

It is herewith described in detail the method of operation of the disposable circuit according to the present disclosure, in a context wherein a patient may be temporarily disconnected from the apparatus 1, thus also from the disposable circuit, during a blood treatment.

In use, before starting with the treatment configuration, the disposable circuit is installed on the apparatus 1; for easing the installation of the disposable circuit, and as well for reducing the risk of errors performed by the qualified operators during said installation, the apparatus 1 may be provided with visual instructions on the GUI helping the operator in the correct placement. The control unit 10 provides on a screen of the user interface of the apparatus 1 selection items for the machine configuration before the treatment. Once prescription and the relevant parameters are entered, the disposable circuit is properly primed; thereafter, the blood circuit is connected to the patient vascular access and the treatment is started.

In case the treatment has to be temporary interrupted, the user interface has a corresponding screen, wherein a selection item, for instance an icon, for the recirculation configuration may be user selected.

As it is briefly disclosed also in figure 24, when the patient has to be disconnected from the apparatus 1, the user selects the recirculation configuration selection item on the screen of the apparatus 1 and then the control unit 10 configures the apparatus 1 in a particular configuration wherein first at least the blood pump 11 and preferably all the pumps of the apparatus 1 are stopped (block 1000). Stopping of at least the blood pump 11 is required for switching or reconfiguring the apparatus 1 to the aforementioned recirculation configuration, and thus the disposable circuit from the treatment configuration to the recirculation configuration and vice-versa, since the blood pump 11 is the pump mainly involved in forcing fluid circulation from the arterial connector to the venous connector in both the operative and in the recirculation configuration.

Albeit in a non-limiting extent, the stopping signal may be sent also to the effluent fluid pump 17 and the dialysis pump 17a. In case dialysis fluid continues circulating in the fluid circuit, at least the ultrafiltration is stopped or set to a minimum very low value. Of course, one or more infusion pumps may run, and the infused fluid be balanced by ultrafiltration.

Then (block 1001) a disconnection of the patient from the apparatus 1 takes place: the user (and/or the apparatus) clamps the blood line ends and then disconnect the arterial connector 40 and venous connector 41 from the access device (e.g., the central catheter or the access needles), so that the arterial connector and the venous connector 40, 41 result both with a respective disconnectable end actually free, ready to be connected to the respective free end of the auxiliary connectors, according to one of the aforementioned embodiments.

Generally, the user disconnects a first connector, normally the arterial connector 40 is firstly disconnected. The disconnected (e.g., arterial) connector is then connected to the first or to the second auxiliary connector, depending on the chosen recirculation configuration. Thereafter, the second connector (e.g., the venous connector 41) is disconnected and subsequently connected to the free connector among the first and the second auxiliary connector defining the recirculation configuration. In other words, the user disconnect and connects the arterial and venous connectors sequentially.

As indicated in block 1002 of figure 24, the first auxiliary connector and the second auxiliary connector are then connected to the venous connector 41 and to the arterial connector 40, preferably according to the disclosure of any of the configurations of the embodiments described before. If the first auxiliary connector and the second auxiliary connectors are of a self-sealing type, there is typically no need for the operator to remove closing caps from the disconnectable ports 70, 70a of the auxiliary connectors; otherwise, before properly setting the connection of the auxiliary connectors to the respective arterial connector 40 and venous connector 41, the operator shall properly remove any protective and/or closing cap from the disconnectable port. Of course, in case the self-sealing connector has no cap, there may be a need to clean/disinfect the self-sealing port before making a connection to it.

It may be noted that flexibility of the conduits of the herein disclosed disposable circuit allow for an easy reconfiguration; it may be further noted that during the switching or reconfiguration from the operative configuration to the recirculation configuration, the circuit is left in place on the apparatus 1. In many cases, the portions of the blood circuit 6, 7 closer to both the arterial connector 40 and venous connector 41 may be the ones which are the more easily accessible and/or free or anyway distant from a position at least temporarily defined on pathways on the apparatus 1: therefore, the step of reciprocal connection of an auxiliary connector with either a venous connector 40 or an arterial connector 41 may be preferably performed by moving a portion of the blood circuit 6, 7, so that is physically the venous connector (or the arterial connector) to be brought first closer and then in contact with the free end port of the respective auxiliary connector. This eases the operation for the user, and further reduces the time required for reconfiguring the circuit and the apparatus. Thanks to this aspect the reconfiguration to the recirculation configuration may be rapidly performed.

Once the proper connection between the first auxiliary connector, the second auxiliary connector and the arterial connector 40 and venous connector 41 is set, the blood lines are unclamped and the operator shall promptly intervene on the user interface of the apparatus 1 to set a command of reactivation of at least the blood pump 11. This step is schematically indicated in block 1003 of figure 24. Preferably this is made through an interaction with a specific icon provided on a screen of the apparatus 1, or through the action on a specific activation button on the apparatus 1 (block 1003, figure 24).

During the recirculation of fluid in the recirculation configuration, it is not wanted that liquids contained in any of the fluid lines connected to the blood circuit 6, 7 are drained therefrom. Thus, according to a recirculation process, any pump of a fluid line connected to the blood circuit 6, 7 is, and rests, deactivated for the entire process of recirculation. This is particularly suitable when the first auxiliary connector and the second auxiliary connector are arranged both downstream the pumps of the fluid lines, because stopping of the pump acts as a means for impeding free fluid circulation in the respective fluid line upstream the pump. This advantage is further enhanced when the pumps acting on the fluid lines are occlusive pumps, like a peristaltic pump, because this latter type of pump not only provides fluid flow additional resistance when not operative, but substantially fully closes the portion of conduct of the fluid line, physically impeding any draining of fluid from the portion upstream thereof. Hence contamination of the fluids contained upstream the pumps in any of the lines introducing fluids in the blood circuit 6, 7 is prevented or anyway significantly reduced.

In a first alternative, when fluid is recirculated in the disposable circuit in said recirculation configuration, the effluent fluid pump 17 and the dialysis pump 17a are stopped; in another and second alternative, when the fluid is recirculated in the disposable circuit in said recirculation configuration, the effluent fluid pump 17 and the dialysis pump 17a are left active (but no ultrafiltration is achieved).

When the temporary disconnection of the patient ends, i.e. when the patient shall be reconnected to the apparatus 1 through the venous connector 40 and the auxiliary connector 41 for the purposes of finishing the temporarily suspended blood treatment, the user selects a specific command on the user interface operatively connected to control unit 10 which stops the activation of the blood pump 11, and if the effluent fluid pump 17 and the dialysis pump 17a were left active during the recirculation of fluid, also those pumps may be stopped. Then the operator clamps and disconnects the venous connector 40 and arterial connector 41 from the respective first auxiliary connector, second auxiliary connector at which they are connected. The closed configuration for the circuit defined when the recirculation configuration of the disposable circuit was started is then ended, and temporarily the disposable circuit is open. If the first auxiliary connector and the second auxiliary connector are not of a self-sealing type, the operator then provides to close the free disconnectable end thereof; otherwise, if the first auxiliary connector and the second auxiliary connector are of a self-sealing type, this latter action of the operator is not required.

The venous connector 40 and arterial connector 41 are then reconnected to the access device of the patient and unclamped; through the menu of the control unit 10 on the screen, the user selects the selection item corresponding to the restart of the blood treatment, and pumps of the apparatus 1, in particular at least the blood pump 11, is then reactivated. Reactivation is preferably made also for the effluent fluid pump 17 and the dialysis pump 17a, and for all infusion pumps (as required by the set treatment mode) for allowing treatment delivery.

It is noted that the above disconnection procedure does not require to restitute blood to the patient before temporary disconnection (though this is also possible). Therefore, recirculation occurring in the blood lines and eventually in the portion of the infusion line involved occurs to patient blood.

As an alternative procedure, extracorporeal blood may be restituted to the patient before recirculation. According to this embodiment, after the blood pump is stopped and the blood withdrawal line 6 is clamped, the arterial connector 40 is disconnected from the access device of the patient and is connected to a bag containing physiological fluid (e.g., saline or replacement fluid). The blood withdrawal line is unclamped and blood pump reactivated to return blood to the patient via the blood return line which is still connected to the vascular access. Once the blood is restituted, blood withdrawal line is clamped and the arterial connector is properly connected to the first or the second auxiliary connector. Then the blood return line is clamped, the venous connector 41 removed from the access device and connected to the other one of the first and second auxiliary connector. The user unclamps the blood lines and starts recirculation of the physiological fluid inside the recirculation circuit.

Notably, in case an infusion line is present on the circuit, the content of the bag feeding the infusion line may be used to provide a suitable fluid for restituting blood. Namely, the blood withdrawal line is clamped and the infusion pump (and if necessary the blood pump, too) is activated to force fluid to enter into the blood lines and push blood to the patient via the blood return line and vascular access. This alternative procedure may be performed even before disconnecting the arterial connector.

During recirculation of fluid the usual alarms, such as the access, arterial and venous pressure alarms are deactivated or the alarm windows changed. Indeed, the different conditions during recirculation is not compatible with the usual alarm thresholds and with no change/deactivation would generate several false alarms.

Parts of the process therein described may be provided through a data processing unit, technically interchangeable with one or more data processing units conceived for executing a portion of a software program or a firmware program loaded to a memory support. The software or firmware program may be written in any known programming language. The electronic computers, if two or more, may be connected each other through a data connection such that the computation powers thereof may be shared; the electronic computers may therefore be installed in different positions thereby realizing the data connection in a distributed computing environment. The data processing unit, or control unit, may be a general purpose processor, specifically configured for executing one or more parts of the process defined in the present disclosure through the software or firmware program, or may be an ASIC or a dedicated processor or an FPGA, specifically programmed for executing at least part of the operations of the process herein described. The memory support may be located inside or outside the processor or control unit or data processing unit, and may be geographically remotely located therefrom.

The invention is not limited to the embodiment/s illustrated in the drawings. Accordingly, it should be understood that where features mentioned in the appended claims are followed by reference signs, such signs are included solely for the purpose of enhancing the intelligibility of the claims and are in no way limiting on the scope of the claims.

It is finally clear that any adaptation or addition is made possible on any part of the elements which form part of the present disclosure without for this departing from the invention defined by the annexed claims.

## Claims

1. A disposable circuit for extracorporeal treatment of blood comprising:
- a filtration unit (2);
- a blood circuit (6, 7) comprising a blood withdrawal line (6) having a first end connected to an inlet of the filtration unit (2), and a blood return line (7) having a first end connected to an outlet of the filtration unit (2), said blood withdrawal line (6) and said blood return line (7) being designed to be connected to a patient cardiovascular system,
wherein the blood withdrawal line (6) has a second end provided with an arterial connector (40) and the blood return line (7) has a second end provided with a venous connector (41), said arterial connector (40) and said venous connector (41) being designed to be detachably connected to a vascular access device of a patient; and wherein said blood circuit is configured to be interfaced with a blood pump (11) for controlling the flow in the blood circuit (6, 7);
- at least one fluid line (15, 21, 25, 42; 42a) connected to the blood circuit (6, 7), the disposable circuit further comprising at least one first auxiliary connector (51, 53, 55, 57, 59) arranged either on the blood circuit (6, 7) or on said at least one fluid line (15, 21, 25, 42; 42a), said at least one first auxiliary connector (51, 53, 55, 57, 59) being configured to be removably connected with at least one between the arterial connector (40) and the venous connector (41) in a recirculation configuration so as to define a closed circuit allowing fluid recirculation in the blood circuit;
**characterized in that**
said disposable circuit further comprises a second auxiliary connector (50, 52, 54, 56, 58) arranged on the blood circuit or on said at least one fluid line (15, 21, 25, 42; 42a), **in that** the first auxiliary connector (51, 53, 55, 57, 59) and
the second auxiliary connector (50, 52, 54, 56, 58) are configured to be removably connected one with the arterial connector (40) and the other with the venous connector (41) so as to define said closed circuit, and **in that** the disposable circuit is reversibly configurable between:
• a treatment configuration, wherein the arterial connector (40) and the venous connector (41) are removably connected to the vascular access device of a patient and blood may be withdrawn from the patient through the blood withdrawal line (6), circulated through the filtration unit (2) and returned to the patient through the blood return line (7), and
• a recirculation configuration, wherein one of the arterial connector (40) and venous connector (41) is removably connected to one of the first auxiliary connector (51, 53, 55, 57, 59) and the second auxiliary connector (50, 52, 54, 56, 58), the other one of the arterial connector (40) and venous connector (41) is removably connected to the other one of the first auxiliary connector (51, 53, 55, 57, 59) and the second auxiliary connector (50, 52, 54, 56, 58), wherein in the recirculation configuration a fluid contained in the blood circuit (6, 7) may be recirculated inside the disposable circuit.

2. A disposable circuit for extracorporeal treatment of blood comprising:
- a filtration unit (2);
- a blood circuit (6, 7) comprising a blood withdrawal line (6) having a first end connected to an inlet of the filtration unit (2), and a blood return line (7) having a first end connected to an outlet of the filtration unit (2), said blood withdrawal line (6) and said blood return line (7) being designed to be connected to a patient cardiovascular system,
wherein the blood withdrawal line (6) has a second end provided with an arterial connector (40) and the blood return line (7) has a second end provided with a venous connector (41), said arterial connector (40) and said venous connector (41) being designed to be detachably connected to a vascular access device of a patient; and wherein said blood circuit is configured to be interfaced with a blood pump (11) for controlling the flow in the blood circuit (6, 7);
wherein the disposable circuit further comprises:
- at least one first auxiliary connector (51, 53, 55, 57, 59) arranged on the blood circuit (6, 7), said at least one first auxiliary connector (51, 53, 55, 57, 59) being configured to be removably connected with at least one connector between the arterial connector (40) and the venous connector (41) in a recirculation configuration so as to define a closed circuit allowing fluid recirculation in the blood circuit; and fl
**characterized in that** the disposable circuit further comprises:
- a second auxiliary connector (50, 52, 54, 56, 58) arranged on the blood circuit, **in that** the first auxiliary connector (51, 53, 55, 57, 59) and the second auxiliary connector (50, 52, 54, 56, 58) are configured to be removably connected one with the arterial connector (40) and the other with the venous connector (41) so as to define said closed circuit, and **in that** the disposable circuit is reversibly configurable between:
• a treatment configuration, wherein the arterial connector (40) and the venous connector (41) are removably connected to the vascular access device of a patient and blood may be withdrawn from the patient through the blood withdrawal line (6), circulated through the filtration unit (2) and returned to the patient through the blood return line (7), and
• a recirculation configuration, wherein one of the arterial connector (40) and venous connector (41) is removably connected to one of the first auxiliary connector (51, 53, 55, 57, 59) and the second auxiliary connector (50, 52, 54, 56, 58), the other one of the arterial connector (40) and venous connector (41) is removably connected to the other one of the first auxiliary connector (51, 53, 55, 57, 59) and the second auxiliary connector (50, 52, 54, 56, 58), wherein in the recirculation configuration a fluid contained in the blood circuit (6, 7) may be recirculated inside the disposable circuit.

3. The disposable circuit of previous claim 1, wherein the at least one fluid line (15, 21, 25, 42; 42a) connected to the blood circuit comprises at least one auxiliary line (42, 42a) and the first auxiliary connector and the second auxiliary connector are installed on the same, single, auxiliary line (42); said auxiliary fluid line (42) being provided with an own fluid pump tract on which a fluid pump is configured to operate, the first auxiliary connector and the second auxiliary connector being installed one upstream with respect to the other.

4. The disposable circuit of any one of the previous claims, wherein at least one connector between the first auxiliary connector (51, 53, 55, 57, 59) and the second auxiliary connector (50, 52, 54, 56, 58), optionally both the first auxiliary connector (51, 53, 55, 57, 59) and the second auxiliary connector (50, 52, 54, 56, 58), is/are three ports connector/s, provided with a disconnectable port (70) configured to be connected to one connector selected among the venous connector (41) and the arterial connector (40),
in particular wherein at least one connector between the first auxiliary connector (51, 53, 55, 57, 59) and the second auxiliary connector (50, 52, 54, 56, 58), optionally both said first auxiliary connector (51, 53, 55, 57, 59) and the second auxiliary connector (50, 52, 54, 56, 58), is/are provided with:
- a first and a second non-disconnectable service port (71) fixed to respective tube portions;
- said at least one free disconnectable port (70).

5. The disposable circuit of any one of the previous claims 1 and 3 to 4, wherein said at least one fluid line (15, 21, 25; 42; 42a) comprise one or more of the following lines:
- a post-blood pump pre-dilution infusion line (15), directly connected to the blood withdrawal line (6);
- a pre-blood pump pre-dilution infusion line (21), directly connected to the blood withdrawal line (6);
- a post-dilution fluid line (25), directly connected to the blood return line (7),
wherein the first auxiliary connector (51, 53, 55, 57, 59) is provided with a first and a second non-disconnectable service port (71) fixed to a respective tube portion of the fluid line (15, 21, 25, 42; 42a) at which the first auxiliary connector is arranged.

6. The disposable circuit of any one of the previous claims 1, and 3 to 5, wherein the second auxiliary connector (50, 52, 54, 56, 58) is provided with a first and a second non-disconnectable service port (71) fixed to a respective tube portion of the fluid line (15, 21, 25, 42; 42a) at which the second auxiliary connector is arranged, in particular, the first auxiliary connector (51, 53, 55, 57, 59) and the second auxiliary connector (50, 52, 54, 56, 58) being fixed to the same fluid line.

7. The disposable circuit of any one of the previous claims 1, and 3 to 7, comprising a pre-blood pump pre-dilution infusion line (21) directly connected to the blood withdrawal line (6) and wherein the first auxiliary connector (53) and the second auxiliary connector (52) are fixed to the a pre-blood pump pre-dilution infusion line (21), wherein in said recirculation configuration the first auxiliary connector (53) arranged on the pre-blood pump pre-dilution infusion line (21) is connected to the arterial connector (40) and the second auxiliary connector (52) arranged on the pre-blood pump pre-dilution infusion line (21) is connected to the venous connector (41).

8. The disposable circuit of previous claim, wherein the first auxiliary connector (53) is arranged on the pre-blood pump pre-dilution infusion line (21) downstream the second auxiliary connector (52).

9. The disposable circuit of any one of the previous claims 1, and 3 to 8, comprising a pre-blood pump pre-dilution infusion line (21) configured to allow fluid administration to the blood circuit downstream the arterial connector (40); said pre-blood pump pre-dilution infusion line (21) comprising a peristaltic pump tract configured to be coupled with a corresponding peristaltic pump and a container optionally containing an anticoagulant solution, for example a citrate solution, said pre-blood pump pre-dilution infusion line (21) being connected to said container, the first auxiliary connector (53), and optionally the second auxiliary connector (52), being connected to the pre-blood pump pre-dilution infusion line (21) downstream the peristaltic pump tract.

10. The disposable circuit of any one of the previous claims, comprising at least one first pressure drop valve or flow resistor (60) configured for even fluid flow distribution during recirculation, said at least one first pressure drop valve or flow resistor (60) being arranged on the blood circuit or on the at least one fluid line (15, 21, 25, 42; 42a), said at least one first pressure drop valve or flow resistor (60) being configured to even respective fluid flow resistances of two connected branch tube portions during recirculation, in particular wherein said at least one first pressure drop valve or flow resistor (60) is arranged in one of the two connected branch tube portions comprising at least one portion of the fluid line (15, 21, 25, 42, 42a) and at least one portion of said blood circuit (6, 7).

11. The disposable circuit of any one of the previous claims, further comprising:
- an effluent fluid line (13) connected to an outlet of the secondary chamber (4), and
- a dialysis fluid line (19) connected to the inlet of the secondary chamber (4),
in particular, the disposable circuit is a CRRT disposable circuit.

12. The disposable circuit of any one of the previous claims 1 to 4, wherein the first auxiliary connector (51, 53, 55, 57, 59) and the second auxiliary connector (50, 52, 54, 56, 58) are arranged on the blood circuit (6, 7), wherein in said recirculation configuration, the first auxiliary connector (59) arranged on the blood circuit (6, 7), in particular on the blood return line (7) is connected to said arterial connector (40) and the second auxiliary connector (58) arranged on the blood circuit (6, 7), in particular on the blood withdrawal line (6), is connected to the venous connector (41).

13. An apparatus for extracorporeal treatment of blood, comprising a disposable circuit according to one or more of claims 1 to 12, said apparatus further comprising a control unit (10) and a blood pump (11) configured to control the flow of blood in said blood circuit (6, 7),
wherein the control unit (10) is configured to:
- stop the blood pump (11);
- receive a command of activation of a fluid recirculation in the blood circuit (6, 7);
- when the disposable circuit is in a recirculation configuration, activate at least the blood pump (11), to cause recirculation of fluid inside the blood circuit (6, 7).

14. The apparatus of claim 13, wherein in the recirculation configuration, the first auxiliary connector (51, 53, 55, 57, 59) is directly connected either with the arterial connector (40) or with the venous connector (41) and the second auxiliary connector (50, 52, 54, 56, 58) is directly connected with the other one of the arterial connector (40) and the venous connector (41), the control unit (10) being configured to maintain the disposable circuit in the recirculation configuration wherein during recirculation either a full flow rate of fluid entering into the first auxiliary connector (51, 53, 55, 57, 59) or into the second auxiliary connector (50, 52, 54, 56, 58) is split into separate partial flow rates or two partial flow rates of fluid entering into the first auxiliary connector (51, 53, 55, 57, 59) or into the second auxiliary connector (50, 52, 54, 56, 58) are merged and exit as a full flow rate.

15. A method of temporary disconnection of a patient from an apparatus for the extracorporeal treatment of blood, the apparatus comprises a disposable circuit according to anyone of the previous claims, the method comprising:
- stopping a blood pump active on the blood circuit;
- disconnecting the arterial connector (40) from the access device of the patient;
- disconnecting the venous connector (41) from the access device of the patient;
- connecting one of the arterial connector (40) and the venous connector (41) to the first auxiliary connector (51, 53, 55, 57, 59) to define a recirculation configuration;
- connecting the other one of the arterial connector (40) and the venous connector (41) to the second auxiliary connector (50, 52, 54, 56, 58) to define a closed circuit in the disposable circuit to define the recirculation configuration;
- running the blood pump to start recirculating fluid inside the closed circuit when the patient remains disconnected from the apparatus.

16. The method of claim 15, further comprising:
- stopping the blood pump to stop recirculating fluid inside the closed circuit;
- disconnecting one connector among the arterial connector (40) and the venous connector (41) from the first auxiliary connector (51, 53, 55, 57, 59);
- optionally disconnecting the other one of the arterial connector (40) and the venous connector (41) from the second auxiliary connector (50, 52, 54, 56, 58);
- reconnecting the arterial connector (40) and the venous connector (41) to the access device of the patient before resuming the treatment,
- reconnecting the venous connector (41) to the access device of the patient before resuming the treatment;
wherein the method steps are performed without changing the disposable circuit and/or disconnecting the disposable circuit from the apparatus.

## Patentansprüche

1. Einwegkreislauf zur extrakorporalen Behandlung von Blut, umfassend:
- eine Filtrationseinheit (2);
- einen Blutkreislauf (6, 7), umfassend eine Blutentnahmeleitung (6), deren erstes Ende mit einem Einlass der Filtrationseinheit (2) verbunden ist, und eine Blutrückführleitung (7), deren erstes Ende mit einem Auslass der Filtrationseinheit (2) verbunden ist, wobei die Blutentnahmeleitung (6) und die Blutrückführleitung (7) zur Verbindung mit einem Herz-Kreislauf-System eines Patienten konzipiert sind,
wobei die Blutentnahmeleitung (6) ein zweites Ende aufweist, das mit einem arteriellen Konnektor (40) ausgestattet ist, und die Blutrückführleitung (7) ein zweites Ende aufweist, das mit einem venösen Konnektor (41) ausgestattet ist, wobei der arterielle Konnektor (40) und der venöse Konnektor (41) zum lösbaren Verbinden mit einer Gefäßzugangvorrichtung eines Patienten konzipiert sind; und wobei der Blutkreislauf ausgestaltet ist, um mit der Blutpumpe (11) über Schnittstelle gekoppelt zu werden, um den Fluss in dem Blutkreislauf (6, 7) zu steuern;
- mindestens eine Fluidleitung (15, 21, 25, 42; 42a), die mit dem Blutkreislauf (6, 7) verbunden ist, wobei der Einwegkreislauf des Weiteren mindestens einen ersten Hilfskonnektor (51, 53, 55, 57, 59) umfasst, der entweder an dem Blutkreislauf (6, 7) oder an der mindestens einen Fluidleitung (15, 21, 25, 42; 42a) angeordnet ist, wobei der mindestens eine erste Hilfskonnektor (51, 53, 55, 57, 59) ausgestaltet ist, um entfernbar mit mindestens einem von dem arteriellen Konnektor (40) und dem venösen Konnektor (41) in einer Rezirkulationskonfiguration verbunden zu werden, um so einen geschlossenen Kreislauf zu definieren, der Fluidrezirkulation in dem Blutkreislauf ermöglicht;
**dadurch gekennzeichnet, dass**
der Einwegkreislauf des Weiteren umfasst:
einen zweiten Hilfskonnektor (50, 52, 54, 56, 58), der an dem Blutkreislauf oder an der mindestens einen Fluidleitung (15, 21, 25, 42; 42a) angeordnet ist, wobei der erste Hilfskonnektor (51, 53, 55, 57, 59) und der zweite Hilfskonnektor (50, 52, 54, 56, 58) so ausgestaltet sind, dass entfernbar einer mit dem arteriellen Konnektor (40) und der andere mit dem venösen Konnektor (41) verbunden wird, um so den geschlossenen Kreislauf zu definieren, und dass der Einwegkreislauf reversibel konfigurierbar ist zwischen:
• einer Behandlungskonfiguration, wobei der arterielle Konnektor (40) und der venöse Konnektor (41) entfernbar mit der Gefäßzugangsvorrichtung eines Patienten verbunden sind und Blut von dem Patienten durch die Blutentnahmeleitung (6) entnommen werden kann, durch die Filtrationseinheit (2) zirkuliert und durch die Blutrückführleitung (7) an den Patienten zurückgeführt werden kann, und
• einer Rezirkulationskonfiguration, wobei einer von dem arteriellen Konnektor (40) und dem venösen Konnektor (41) entfernbar mit einem von dem ersten Hilfskonnektor (51, 53, 55, 57, 59) und dem zweiten Hilfskonnektor (50, 52, 54, 56, 58) verbunden ist, der andere von dem arteriellen Konnektor (40) und dem venösen Konnektor (41) entfernbar mit dem anderen von dem ersten Hilfskonnektor (51, 53, 55, 57, 59) und dem zweiten Hilfskonnektor (50, 52, 54, 56, 58) verbunden ist, wobei in der Rezirkulationskonfiguration ein in dem Blutkreislauf (6, 7) enthaltenes Fluid innerhalb des Einwegkreislaufs rezirkuliert werden kann.

2. Einwegkreislauf zur extrakorporalen Behandlung von Blut, umfassend:
- eine Filtrationseinheit (2);
- einen Blutkreislauf (6, 7), umfassend eine Blutentnahmeleitung (6), deren erstes Ende mit einem Einlass der Filtrationseinheit (2) verbunden ist, und eine Blutrückführleitung (7), deren erstes Ende mit einem Auslass der Filtrationseinheit (2) verbunden ist, wobei die Blutentnahmeleitung (6) und die Blutrückführleitung (7) zur Verbindung mit einem Herz-Kreislauf-System eines Patienten konzipiert sind,
wobei die Blutentnahmeleitung (6) ein zweites Ende aufweist, das mit einem arteriellen Konnektor (40) ausgestattet ist, und die Blutrückführleitung (7) ein zweites Ende aufweist, das mit einem venösen Konnektor (41) ausgestattet ist, wobei der arterielle Konnektor (40) und der venöse Konnektor (41) zum lösbaren Verbinden mit einer Gefäßzugangvorrichtung eines Patienten konzipiert sind; und wobei der Blutkreislauf ausgestaltet ist, um mit der Blutpumpe (11) gekoppelt zu werden, um den Fluss in dem Blutkreislauf (6, 7) zu steuern;
wobei der Einwegkreislauf des Weiteren umfasst:
- mindestens einen ersten Hilfskonnektor (51, 53, 55, 57, 59), der an dem Blutkreislauf (6, 7) angeordnet ist, wobei mindestens ein erster Hilfskonnektor (51, 53, 55, 57, 59) ausgestaltet ist, um entfernbar mit mindestens einem Konnektor von dem arteriellen Konnektor (40) und dem venösen Konnektor (41) in einer Rezirkulationskonfiguration verbunden zu werden, um so einen geschlossenen Kreislauf zu definieren, der Fluidrezirkulation in dem Blutkreislauf ermöglicht; und
**dadurch gekennzeichnet, dass** der Einwegkreislauf des Weiteren umfasst:
- einen zweiten Hilfskonnektor (50, 52, 54, 56, 58), der an dem Blutkreislauf angeordnet ist, wobei der erste Hilfskonnektor (51, 53, 55, 57, 59) und der zweite Hilfskonnektor (50, 52, 54, 56, 58) so ausgestaltet sind, dass entfernbar einer mit dem arteriellen Konnektor (40) und der andere mit dem venösen Konnektor (41) verbunden wird, um so den geschlossenen Kreislauf zu definieren,
und dass der Einwegkreislauf reversibel konfigurierbar ist zwischen:
• einer Behandlungskonfiguration, wobei der arterielle Konnektor (40) und der venöse Konnektor (41) entfernbar mit der Gefäßzugangsvorrichtung eines Patienten verbunden sind und Blut von dem Patienten durch die Blutentnahmeleitung (6) entnommen werden kann, durch die Filtrationseinheit (2) zirkuliert und durch die Blutrückführleitung (7) an den Patienten zurückgeführt werden kann, und
• einer Rezirkulationskonfiguration, wobei einer von dem arteriellen Konnektor (40) und dem venösen Konnektor (41) entfernbar mit einem von dem ersten Hilfskonnektor (51, 53, 55, 57, 59) und dem zweiten Hilfskonnektor (50, 52, 54, 56, 58) verbunden ist, der andere von dem arteriellen Konnektor (40) und dem venösen Konnektor (41) entfernbar mit dem anderen von dem ersten Hilfskonnektor (51, 53, 55, 57, 59) und dem zweiten Hilfskonnektor (50, 52, 54, 56, 58) verbunden ist, wobei in der Rezirkulationskonfiguration ein in dem Blutkreislauf (6, 7) enthaltenes Fluid innerhalb des Einwegkreislaufs rezirkuliert werden kann.

3. Einwegkreislauf nach dem vorhergehenden Anspruch 1, wobei die mindestens eine Fluidleitung (15, 21, 25, 42; 42a), die mit dem Blutkreislauf verbunden ist, mindestens eine Hilfsleitung (42, 42a) umfasst, und der erste Hilfskonnektor und der zweite Hilfskonnektor an der gleichen einzelnen Hilfsleitung (42) installiert sind; wobei die Hilfsfluidleitung (42) mit einem eigenen Fluidpumpentrakt ausgestattet ist, an dem eine Fluidpumpe konfigurationsgemäß betrieben wird, wobei der erste Hilfskonnektor und der zweite Hilfskonnektor einer stromaufwärts zu dem anderen installiert sind.

4. Einwegkreislauf nach einem der vorhergehenden Ansprüche, wobei mindestens ein Konnektor von dem ersten Hilfskonnektor (51, 53, 55, 57, 59) und dem zweiten Hilfskonnektor (50, 52, 54, 56, 58), gegebenenfalls sowohl der erste Hilfskonnektor (51, 53, 55, 57, 59) als auch der zweite Hilfskonnektor (50, 52, 54, 56, 58), Konnektor(en) mit drei Anschlüssen ist/sind, der/die mit einem trennbaren Anschluss (70) ausgestattet ist/sind, der ausgestaltet ist, um mit einem Konnektor ausgewählt aus dem venösen Konnektor (41) und dem arteriellen Konnektor (40) verbunden zu werden,
wobei insbesondere mindestens ein Konnektor von dem ersten Hilfskonnektor (51, 53, 55, 57, 59) und dem zweiten Hilfskonnektor (50, 52, 54, 56, 58), gegebenenfalls sowohl der erste Hilfskonnektor (51, 53, 55, 57, 59) als auch der zweite Hilfskonnektor (50, 52, 54, 56, 58), ausgestattet ist/sind mit:
- einem ersten und einem zweiten nicht-trennbaren Serviceanschluss (71), angebracht an jeweiligen Schlauchabschnitten;
- dem mindestens einen freien trennbaren Anschluss (70) .

5. Einwegkreislauf nach einem der vorhergehenden Ansprüche 1 und 3 bis 4, wobei die mindestens eine Fluidleitung (15, 21, 25; 42; 42a) eine oder mehrere der folgenden Leitungen umfasst:
- eine Nachblutpumpen-Vorverdünnungs-Infusionsleitung (15), die direkt mit der Blutentnahmeleitung (6) verbunden ist;
- eine Vorblutpumpen-Vorverdünnungs-Infusionsleitung (21), die direkt mit der Blutentnahmeleitung (6) verbunden ist;
- eine Nachverdünnungs-Fluidleitung (25), die direkt mit der Blutrückführleitung (7) verbunden ist,
wobei der erste Hilfskonnektor (51, 53, 55, 57, 59) mit einem ersten Ende und einem zweiten nicht-trennbaren Serviceanschluss (71) ausgestattet ist, angebracht an einem jeweiligen Schlauchabschnitt der Fluidleitung (15, 21, 25, 42; 42a), an dem der erste Hilfskonnektor angeordnet ist.

6. Einwegkreislauf nach einem der vorhergehenden Ansprüche 1 und 3 bis 5, wobei der zweite Hilfskonnektor (50, 52, 54, 56, 58) mit einem ersten und einem zweiten nicht-trennbaren Serviceanschluss (71), angebracht an einem jeweiligen Schlauchabschnitt der Fluidleitung (15, 21, 25, 42; 42a), an dem der zweite Hilfskonnektor angeordnet ist, ausgestattet ist, wobei insbesondere der erste Hilfskonnektor (51, 53, 55, 57, 59) und der zweite Hilfskonnektor (50, 52, 54, 56, 58) an der gleichen Fluidleitung angebracht sind.

7. Einwegkreislauf nach einem der vorhergehenden Ansprüche 1 und 3 bis 7, umfassend eine Vorblutpumpen-Vorverdünnungs-Infusionsleitung (21), die direkt mit einer Blutentnahmeleitung (6) verbunden ist, und wobei der erste Hilfskonnektor (53) und der zweite Hilfskonnektor (52) an der Vorblutpumpen-Vorverdünnungs-Infusionsleitung (21) angebracht sind, wobei in der Rezirkulationskonfiguration der erste Hilfskonnektor (53), der an der Vorblutpumpen-Vorverdünnungs-Infusionsleitung (21) angeordnet ist, mit dem arteriellen Konnektor (40) verbunden ist, und der zweite Hilfskonnektor (52), der an der Vorblutpumpen-Vorverdünnungs-Infusionsleitung (21) angeordnet ist, mit dem venösen Konnektor (41) verbunden ist.

8. Einwegkreislauf nach einem der vorhergehenden Ansprüche, wobei der erste Hilfskonnektor (53) an der Vorblutpumpen-Vorverdünnungs-Infusionsleitung (21) stromabwärts von dem zweiten Hilfskonnektor (52) angeordnet ist.

9. Einwegkreislauf nach einem der vorhergehenden Ansprüche 1 und 3 bis 8, umfassend eine Vorblutpumpen-Vorverdünnungs-Infusionsleitung (21), die ausgestaltet ist, um Fluidverabreichung an den Blutkreislauf stromabwärts des arteriellen Konnektors (40) zu ermöglichen; wobei die Vorblutpumpen-Vorverdünnungs-Infusionsleitung (21) einen peristaltischen Pumpentrakt umfasst, der ausgestaltet ist, um mit einer entsprechenden peristaltischen Pumpe und einem Behälter gekoppelt zu werden, der gegebenenfalls eine Antikoagulanslösung enthält, beispielsweise eine Citratlösung, wobei die Vorblutpumpen-Vorverdünnungs-Infusionsleitung (21) mit dem Behälter verbunden ist, der erste Hilfskonnektor (53) und gegebenenfalls der zweite Hilfskonnektor (52) mit der Vorblutpumpen-Vorverdünnungs-Infusionsleitung (212) stromabwärts des peristaltischen Pumpentrakts verbunden ist/sind.

10. Einwegkreislauf nach einem der vorhergehenden Ansprüche, umfassend mindestens ein erstes Druckabfallventil oder einen ersten Flusswiderstand (60), das/der zur gleichförmigen Fluidflussverteilung während der Rezirkulation ausgestaltet ist, wobei das mindestens eine erste Druckabfallventil oder der erste Flusswiderstand (60) an dem Blutkreislauf oder an der mindestens einen Fluidleitung (15, 21, 25, 42; 42a) angeordnet ist, wobei das mindestens eine erste Druckabfallventil oder der erste Flusswiderstand (60) ausgestaltet ist, um jeweilige Fluidflusswiderstände von zwei verbundenen Zweigschlauchabschnitten während der Rezirkulation zu egalisieren, wobei insbesondere das mindestens eine erste Druckabfallventil oder der erste Flusswiderstand (60) in einem der zwei verbundenen Zweigschlauchabschnitte angeordnet ist, der mindestens einen Abschnitt der Fluidleitung (15, 21, 25, 42, 42a) und mindestens einen Abschnitt des Blutkreislaufs (6, 7) umfasst.

11. Einwegkreislauf nach einem der vorhergehenden Ansprüche, des Weiteren umfassend:
- eine Abflussfluidleitung (13), die mit einem Auslass der Sekundärkammer (4) verbunden ist, und
- eine Dialysefluidleitung (19), die mit dem Einlass der Sekundärkammer (4) verbunden ist, wobei der Einwegkreislauf insbesondere ein CRRT-Einwegkreislauf ist.

12. Einwegkreislauf nach einem der vorhergehenden Ansprüche 1 bis 4, wobei der erste Hilfskonnektor (51, 53, 55, 57, 59) und der zweite Hilfskonnektor (50, 52, 54, 56, 58) an dem Blutkreislauf (6, 7) angeordnet sind, wobei in der Rezirkulationskonfiguration der erste Hilfskonnektor (59), der an dem Blutkreislauf (6, 7), insbesondere an der Blutrückführleitung (7) angeordnet ist, mit dem arteriellen Konnektor (40) verbunden ist, und der zweite Hilfskonnektor (58), der an dem Blutkreislauf (6, 7), insbesondere an der Blutentnahmeleitung (6) angeordnet ist, mit dem venösen Konnektor (41) verbunden ist.

13. Gerät zur extrakorporalen Behandlung von Blut, umfassend einen Einwegkreislauf nach einem oder mehreren der Ansprüche 1 bis 12, wobei das Gerät des Weiteren eine Steuereinheit (10) und eine Blutpumpe (11) umfasst, die ausgestaltet sind, um den Fluss von Blut in dem Blutkreislauf (6, 7) zu steuern,
wobei die Steuereinheit (10) ausgestaltet ist zum:
- Stoppen der Blutpumpe (11);
- Empfangen eines Aktivierungsbefehls einer Fluidrezirkulation in dem Blutkreislauf (6, 7);
- wenn der Einwegkreislauf in einer Rezirkulationskonfiguration vorliegt, Aktivieren von mindestens der Blutpumpe (11), um Rezirkulation von Fluid innerhalb des Blutkreislaufs (6, 7) zu bewirken.

14. Gerät nach Anspruch 13, wobei in der Rezirkulationskonfiguration der erste Hilfskonnektor (51, 53, 55, 57, 59) direkt entweder mit dem arteriellen Konnektor (40) oder mit dem venösen Konnektor (41) verbunden ist, und der zweite Hilfskonnektor (50, 52, 54, 56, 58) direkt mit dem anderen von dem arteriellen Konnektor (40) und dem venösen Konnektor (41) verbunden ist, die Steuereinheit (10) ausgestaltet ist, um den Einwegkreislauf in der Rezirkulationskonfiguration zu halten, wobei während der Rezirkulation entweder eine volle Flussrate an Fluid, die in den ersten Hilfskonnektor (51, 53, 55, 57, 59) oder in den zweiten Hilfskonnektor (50, 52, 54, 56, 58) eintritt, in separate Teilflussraten aufgeteilt wird, oder zwei Teilflussraten an Fluid, die in den ersten Hilfskonnektor (51, 53, 55, 57, 59) oder in den zweiten Hilfskonnektor (50, 52, 54, 56, 58) eintreten, zusammengeführt werden und als volle Flussrate austreten.

15. Verfahren zum zeitweiligen Trennen eines Patienten von einem Gerät zur extrakorporalen Behandlung von Blut, wobei das Gerät einen Einwegkreislauf gemäß einem der vorhergehenden Ansprüche umfasst, wobei das Verfahren umfasst:
- Stoppen einer Blutpumpe, die auf den Blutkreislauf einwirkt;
- Trennen des arteriellen Konnektors (40) von der Zugangsvorrichtung des Patienten;
- Trennen des venösen Konnektors (41) von der Zugangsvorrichtung des Patienten;
- Verbinden von einem von dem arteriellen Konnektor (40) und dem venösen Konnektor (41) mit dem ersten Hilfskonnektor (51, 53, 55, 57, 59), um eine Rezirkulationskonfiguration zu definieren;
- Verbinden von dem anderen von dem arteriellen Konnektor (40) und dem venösen Konnektor (41) mit dem zweiten Hilfskonnektor (50, 52, 54, 56, 58), um einen geschlossenen Kreislauf in dem Einwegkreislauf zu definieren, um die Rezirkulationskonfiguration zu definieren;
- Laufenlassen der Blutpumpe, um mit dem Rezirkulieren von Fluid innerhalb des geschlossenen Kreislaufs zu beginnen, während der Patient von dem Gerät getrennt bleibt.

16. Verfahren nach Anspruch 15, des Weiteren umfassend:
- Stoppen der Blutpumpe, um das Rezirkulieren von Fluid innerhalb des geschlossenen Kreislaufs zu stoppen;
- Trennen eines Konnektors aus dem arteriellen Konnektor (40) und dem venösen Konnektor (41) von dem ersten Hilfskonnektor (51, 53, 55, 57, 59);
- gegebenenfalls Trennen des anderen aus dem arteriellen Konnektor (40) und dem venösen Konnektor (41) von dem zweiten Hilfskonnektor (50, 52, 54, 56, 58);
- erneutes Verbinden des arteriellen Konnektors (40) und des venösen Konnektors (41) mit der Zugangsvorrichtung des Patienten, bevor die Behandlung wieder aufgenommen wird,
- erneutes Verbinden des venösen Konnektors (41) mit der Zugangsvorrichtung des Patienten, bevor die Behandlung wieder aufgenommen wird;
- wobei die Verfahrensschritte durchgeführt werden, ohne den Einwegkreislauf zu wechseln und/oder den Einwegkreislauf von dem Gerät zu trennen.

## Revendications

1. Circuit jetable destiné à un traitement extracorporel du sang comprenant :
- une unité de filtration (2) ;
- un circuit sanguin (6, 7) comprenant une ligne de prélèvement sanguin (6) ayant une première extrémité raccordée à une entrée de l'unité de filtration (2), et une ligne de retour sanguin (7) ayant une première extrémité raccordée à une sortie de l'unité de filtration (2), ladite ligne de prélèvement sanguin (6) et ladite ligne de retour sanguin (7) étant conçues pour être raccordées au système cardiovasculaire d'un patient, dans lequel la ligne de prélèvement sanguin (6) a une seconde extrémité pourvue d'un raccord artériel (40) et la ligne de retour sanguin (7) a une seconde extrémité pourvue d'un raccord veineux (41), ledit raccord artériel (40) et ledit raccord veineux (41) étant conçus pour être raccordés de façon amovible à un dispositif d'accès vasculaire d'un patient ; et dans lequel ledit circuit sanguin est configuré pour assurer l'interface avec une pompe à sang (11) afin de réguler le débit dans le circuit sanguin (6, 7) ;
- au moins une ligne de fluide (15, 21, 25, 42 ; 42a) raccordée au circuit sanguin (6, 7), le circuit jetable comprenant en outre au moins un premier raccord auxiliaire (51, 53, 55, 57, 59) agencé soit sur le circuit sanguin (6, 7), soit sur ladite au moins une ligne de fluide (15, 21, 25, 42 ; 42a), ledit au moins un premier raccord auxiliaire (51, 53, 55, 57, 59) étant configuré pour être raccordé de façon amovible à au moins un entre le raccord artériel (40) et le raccord veineux (41) dans une configuration de recirculation de façon à définir un circuit fermé permettant la recirculation de fluide dans le circuit sanguin ;
**caractérisé en ce que**
ledit circuit jetable comprend en outre un second raccord auxiliaire (50, 52, 54, 56, 58) agencé sur le circuit sanguin ou sur ladite au moins une ligne de fluide (15, 21, 25, 42 ; 42a), **en ce que** le premier raccord auxiliaire (51, 53, 55, 57, 59) et le second raccord auxiliaire (50, 52, 54, 56, 58) sont configurés pour être raccordés de façon amovible l'un au raccord artériel (40) et l'autre au raccord veineux (41) de façon à définir ledit circuit fermé,
et **en ce que** le circuit jetable est configurable de façon réversible entre :
• une configuration de traitement, dans laquelle le raccord artériel (40) et le raccord veineux (41) sont raccordés de façon amovible au dispositif d'accès vasculaire d'un patient et le sang peut être prélevé sur le patient par le biais de la ligne de prélèvement sanguin (6), mis en circulation à travers l'unité de filtration (2) et renvoyé au patient par le biais de la ligne de retour sanguin (7), et
• une configuration de recirculation, dans laquelle l'un du raccord artériel (40) et du raccord veineux (41) est raccordé de façon amovible à l'un du premier raccord auxiliaire (51, 53, 55, 57, 59) et du second raccord auxiliaire (50, 52, 54, 56, 58), l'autre du raccord artériel (40) et du raccord veineux (41) est raccordé de façon amovible à l'autre du premier raccord auxiliaire (51), 53, 55, 57, 59) et du second raccord auxiliaire (50, 52, 54, 56, 58), dans lequel, dans la configuration de recirculation, un fluide contenu dans le circuit sanguin (6, 7) peut être remis en circulation à l'intérieur du circuit jetable.

2. Circuit jetable destiné à un traitement extracorporel du sang comprenant :
- une unité de filtration (2) ;
- un circuit sanguin (6, 7) comprenant une ligne de prélèvement sanguin (6) ayant une première extrémité raccordée à une entrée de l'unité de filtration (2), et une ligne de retour sanguin (7) ayant une première extrémité raccordée à une sortie de l'unité de filtration (2), ladite ligne de prélèvement sanguin (6) et ladite ligne de retour sanguin (7) étant conçues pour être raccordées au système cardiovasculaire d'un patient, dans lequel la ligne de prélèvement sanguin (6) a une seconde extrémité pourvue d'un raccord artériel (40) et la ligne de retour sanguin (7) a une seconde extrémité pourvue d'un raccord veineux (41), ledit raccord artériel (40) et ledit raccord veineux (41) étant conçus pour être raccordés de façon amovible à un dispositif d'accès vasculaire d'un patient ; et dans lequel ledit circuit sanguin est configuré pour assurer l'interface avec une pompe à sang (11) afin de réguler le débit dans le circuit sanguin (6, 7) ;
dans lequel le circuit jetable comprend en outre :
- au moins un premier raccord auxiliaire (51, 53, 55, 57, 59) agencé sur le circuit sanguin (6, 7), ledit au moins un premier raccord auxiliaire (51, 53, 55, 57, 59) étant configuré pour être relié de façon amovible à au moins un raccord entre le raccord artériel (40) et le raccord veineux (41) dans une configuration de recirculation de façon à définir un circuit fermé permettant une recirculation de fluide dans le circuit sanguin ; et **caractérisé en ce que** le circuit jetable comprend en outre :
- un second raccord auxiliaire (50, 52, 54, 56, 58) agencé sur le circuit sanguin, **en ce que** le premier raccord auxiliaire (51, 53, 55, 57, 59) et le second raccord auxiliaire (50, 52, 54, 56, 58) sont configurés pour être raccordés de façon amovible l'un avec le raccord artériel (40) et l'autre avec le raccord veineux (41) de façon à définir ledit circuit fermé,
et **en ce que** le circuit jetable est configurable de façon réversible entre :
• une configuration de traitement, dans laquelle le raccord artériel (40) et le raccord veineux (41) sont raccordés de façon amovible au dispositif d'accès vasculaire d'un patient et le sang peut être prélevé sur le patient par le biais de la ligne de prélèvement sanguin (6), mis en circulation à travers l'unité de filtration (2) et renvoyé au patient par le biais de la ligne de retour sanguin (7), et
• une configuration de recirculation, dans laquelle l'un du raccord artériel (40) et du raccord veineux (41) est raccordé de façon amovible à l'un du premier raccord auxiliaire (51, 53, 55, 57, 59) et du second raccord auxiliaire (50, 52, 54, 56, 58), l'autre du raccord artériel (40) et du raccord veineux (41) est raccordé de façon amovible à l'autre du premier raccord auxiliaire (51, 53, 55, 57, 59) et du second raccord auxiliaire (50, 52, 54, 56, 58), dans lequel, dans la configuration de recirculation, un fluide contenu dans le circuit sanguin (6, 7) peut être remis en circulation à l'intérieur du circuit jetable.

3. Circuit jetable selon la revendication 1 précédente, dans lequel l'au moins une ligne de fluide (15, 21, 25, 42 ; 42a) raccordée au circuit sanguin comprend au moins une ligne auxiliaire (42, 42a) et le premier raccord auxiliaire et le second raccord auxiliaire sont installés sur la même ligne auxiliaire unique (42) ; ladite ligne de fluide auxiliaire (42) étant pourvue d'un conduit de pompe à fluide propre sur lequel une pompe à fluide est configurée pour fonctionner, le premier raccord auxiliaire et le second raccord auxiliaire étant installés l'un en amont par rapport à l'autre.

4. Circuit jetable selon l'une quelconque des revendications précédentes, dans lequel au moins un raccord entre le premier raccord auxiliaire (51, 53, 55, 57, 59) et le second raccord auxiliaire (50, 52, 54, 56, 58), éventuellement à la fois le premier raccord auxiliaire (51, 53, 55, 57, 59) et le second raccord auxiliaire (50, 52, 54, 56, 58), est/sont un/des raccord(s) à trois orifices, pourvus d'un orifice débranchable (70) configuré pour être raccordé à un raccord choisi parmi le raccord veineux (41) et le raccord artériel (40),
en particulier dans lequel au moins un raccord entre le premier raccord auxiliaire (51, 53, 55, 57, 59) et le second raccord auxiliaire (50, 52, 54, 56, 58), éventuellement à la fois ledit premier raccord auxiliaire (51, 53, 55, 57, 59) et le second raccord auxiliaire (50, 52, 54, 56, 58), est/sont pourvu(s) de :
- un premier et un second orifice de service non débranchable (71) fixés à des parties de tube respectives ;
- ledit au moins un orifice débranchable libre (70).

5. Circuit jetable selon l'une quelconque des revendications 1 et 3 à 4 précédentes, dans lequel ladite au moins une ligne de fluide (15, 21, 25 ; 42 ; 42a) comprend une ou plusieurs des lignes suivantes :
- une ligne de perfusion de prédilution post-pompe à sang (15), raccordée directement à la ligne de prélèvement sanguin (6) ;
- une ligne de perfusion de prédilution prépompe à sang (21), raccordée directement à la ligne de prélèvement sanguin (6) ;
- une ligne de fluide de post-dilution (25), raccordée directement à la ligne de retour sanguin (7), dans lequel le premier raccord auxiliaire (51, 53, 55, 57, 59) est pourvu d'un premier et d'un second orifice de service non débranchable (71) fixé à une partie de tube respective de la ligne de fluide (15, 21, 25, 42 ; 42a) au niveau de laquelle est agencé le premier raccord auxiliaire.

6. Circuit jetable selon l'une quelconque des revendications précédentes 1, et 3 à 5, dans lequel le second raccord auxiliaire (50, 52, 54, 56, 58) est pourvu d'un premier et d'un second orifice de service non débranchable (71) fixé à une partie de tube respective de la ligne de fluide (15, 21, 25, 42 ; 42a) au niveau de laquelle est agencé le second raccord auxiliaire, en particulier, le premier raccord auxiliaire (51, 53, 55, 57, 59) et le second raccord auxiliaire (50, 52, 54, 56, 58) étant fixés à la même ligne de fluide.

7. Circuit jetable selon l'une quelconque des revendications précédentes 1, et 3 à 7, comprenant une ligne de perfusion de prédilution prépompe à sang (21) directement raccordée à la ligne de prélèvement sanguin (6) et dans lequel le premier raccord auxiliaire (53) et le second raccord auxiliaire (52) sont fixés à la ligne de perfusion de prédilution prépompe à sang (21), dans lequel, dans ladite configuration de recirculation, le premier raccord auxiliaire (53) agencé sur la ligne de perfusion de prédilution prépompe à sang (21) est raccordé au raccord artériel (40) et le second raccord auxiliaire (52) agencé sur la ligne de perfusion de prédilution prépompe à sang (21) est raccordé au raccord veineux (41).

8. Circuit jetable selon la revendication précédente, dans lequel le premier raccord auxiliaire (53) est agencé sur la ligne de perfusion de prédilution prépompe à sang (21) en aval du second raccord auxiliaire (52).

9. Circuit jetable selon l'une quelconque des revendications précédentes 1, et 3 à 8, comprenant une ligne de perfusion de prédilution prépompe à sang (21) configurée pour permettre l'administration de fluide au circuit sanguin en aval du raccord artériel (40) ; ladite ligne de perfusion de prédilution prépompe à sang (21) comprenant un conduit de pompe péristaltique configuré pour être accouplé à une pompe péristaltique correspondante et à un récipient contenant éventuellement une solution anticoagulante, par exemple une solution de citrate, ladite ligne de perfusion de prédilution prépompe à sang (21) étant raccordée audit récipient, le premier raccord auxiliaire (53), et éventuellement le second raccord auxiliaire (52), étant raccordés à la ligne de perfusion de prédilution prépompe à sang (21) en aval du conduit de pompe péristaltique.

10. Circuit jetable selon l'une quelconque des revendications précédentes, comprenant au moins une première soupape de chute de pression ou résistance à l'écoulement (60) configurée pour une uniformiser une distribution d'écoulement de fluide pendant la recirculation, ladite au moins une première soupape de chute de pression ou résistance à l'écoulement (60) étant agencée sur le circuit sanguin ou sur l'au moins une ligne de fluide (15, 21, 25, 42 ; 42a), ladite au moins une première soupape de chute de pression ou résistance à l'écoulement (60) étant configurée pour uniformiser des résistances respectives à l'écoulement de fluide de deux parties de tube de dérivation raccordées pendant la recirculation, en particulier dans lequel ladite au moins une première soupape de chute de pression ou résistance à l'écoulement (60) est agencée dans l'une des deux parties de tube de dérivation raccordées comprenant au moins une partie de la ligne de fluide (15, 21, 25, 42, 42a) et au moins une partie dudit circuit sanguin (6, 7).

11. Circuit jetable selon l'une quelconque des revendications précédentes, comprenant en outre :
- une ligne de fluide effluent (13) raccordée à une sortie de la chambre secondaire (4), et
- une ligne de fluide de dialyse (19) raccordée à l'entrée de la chambre secondaire (4),
en particulier, le circuit jetable est un circuit jetable CRRT.

12. Circuit jetable selon l'une quelconque des revendications 1 à 4 précédentes, dans lequel le premier raccord auxiliaire (51, 53, 55, 57, 59) et le second raccord auxiliaire (50, 52, 54, 56, 58) sont agencés sur le circuit sanguin (6, 7), dans lequel, dans ladite configuration de recirculation, le premier raccord auxiliaire (59) agencé sur le circuit sanguin (6, 7), en particulier sur la ligne de retour sanguin (7) est raccordé audit raccord artériel (40) et le second raccord auxiliaire (58) agencé sur le circuit sanguin (6, 7), en particulier sur la ligne de prélèvement sanguin (6), est raccordé au raccord veineux (41).

13. Appareil destiné à un traitement extracorporel du sang, comprenant un circuit jetable selon une ou plusieurs des revendications 1 à 12, ledit appareil comprenant en outre une unité de commande (10) et une pompe à sang (11) configurées pour réguler le flux sanguin dans ledit circuit sanguin (6, 7),
dans lequel l'unité de commande (10) est configurée pour :
- arrêter la pompe à sang (11) ;
- recevoir une commande d'activation d'une recirculation de fluide dans le circuit sanguin (6, 7) ;
- lorsque le circuit jetable est dans une configuration de recirculation, activer au moins la pompe à sang (11), pour provoquer une recirculation du fluide à l'intérieur du circuit sanguin (6, 7).

14. Appareil selon la revendication 13, dans lequel, dans la configuration de recirculation, le premier raccord auxiliaire (51, 53, 55, 57, 59) est directement raccordé soit au raccord artériel (40), soit au raccord veineux (41) et le second raccord auxiliaire (50, 52, 54, 56, 58) est directement raccordé à l'autre du raccord artériel (40) et du raccord veineux (41), l'unité de commande (10) étant configurée pour maintenir le circuit jetable dans la configuration de recirculation dans laquelle, pendant la recirculation, soit un débit total de fluide entrant dans le premier raccord auxiliaire (51, 53, 55, 57, 59) ou dans le second raccord auxiliaire (50, 52, 54, 56, 58) est divisé en deux débits partiels distincts, soit deux débits partiels de fluide entrant dans le premier raccord auxiliaire (51, 53, 55, 57, 59) ou dans le second raccord auxiliaire (50, 52, 54, 56, 58) sont fusionnés et sortent en tant que débit total.

15. Procédé de débranchement temporaire d'un patient d'un appareil de traitement extracorporel du sang, l'appareil comprenant un circuit jetable selon l'une quelconque des revendications précédentes, le procédé comprenant :
- l'arrêt d'une pompe à sang active sur le circuit sanguin ;
- le débranchement du raccord artériel (40) du dispositif d'accès du patient ;
- le débranchement du raccord veineux (41) du dispositif d'accès du patient ;
- le raccordement de l'un du raccord artériel (40) et du raccord veineux (41) au premier raccord auxiliaire (51, 53, 55, 57, 59) pour définir une configuration de recirculation ;
- le raccordement de l'autre du raccord artériel (40) et du raccord veineux (41) au second raccord auxiliaire (50, 52, 54, 56, 58) pour définir un circuit fermé dans le circuit jetable pour définir la configuration de recirculation ;
- le fonctionnement de la pompe à sang pour commencer à faire recirculer le fluide à l'intérieur du circuit fermé lorsque le patient reste débranché de l'appareil.

16. Procédé selon la revendication 15, comprenant en outre :
- l'arrêt de la pompe à sang pour arrêter la recirculation du fluide à l'intérieur du circuit fermé ;
- le débranchement d'un raccord parmi le raccord artériel (40) et le raccord veineux (41) du premier raccord auxiliaire (51, 53, 55, 57, 59) ;
- éventuellement le débranchement de l'autre du raccord artériel (40) et du raccord veineux (41) du second raccord auxiliaire (50, 52, 54, 56, 58) ;
- le nouveau raccordement du raccord artériel (40) et du raccord veineux (41) au dispositif d'accès du patient avant de reprendre le traitement,
- le nouveau raccordement du raccord veineux (41) au dispositif d'accès du patient avant de reprendre le traitement ;
dans lequel les étapes de procédé sont effectuées sans changer le circuit jetable et/ou débrancher le circuit jetable de l'appareil.
